# EUROPEAN PATENT APPLICATION

(11) **EP 4 737 590 A1**
(43) Date of publication of application: **06.05.2026**
(21) Application number: 24210236.6
(22) Date of filing: 31.10.2024
(51) Int. Cl.: C12Q 1/6886

(54) **METHOD FOR CLASSIFYING A CUTANEOUS MELANOMA PATIENT REGARDING ITS RISK OF DEVELOPING METASTASES**

(71) Applicant: Eberhard Karls Universität Tübingen (Medizinische Fakultät), 72074 Tübingen (DE)
(72) Inventor: Röcken, Martin, 72074 Tübingen (DE); Sinnberg, Tobias, 72072 Tübingen (DE); Chtziioannou, Eftychia, 72076 Tübingen (DE); Armeanu-Ebinger, Sorin, 72076 Tübingen (DE); Schroeder, Christopher, 72076 Tübingen (DE); Riess, Olaf, 72070 Tübingen (DE); Ossowski, Stephan, 70184 Stuttgart (DE)
(74) Representative: Witte, Weller & Partner Patentanwälte mbB

(57) **Abstract**

The present invention relates to a method ex *vivo* for classifying a cutaneous melanoma patient, regarding its risk of developing metastases. It also relates to the use of a marker gene which underwent a somatic copy number alteration (SCNA) for classifying a cutaneous melanoma patient regarding its risk of developing metastases. Furthermore, it relates to a method *ex vivo* for identifying treatment targets in a cutaneous melanoma patient. The invention also relates to a method of selecting a treatment of a cutaneous melanoma patient.

## Description

### FIELD OF THE INVENTION

The present invention relates to a method ex *vivo* for classifying a cutaneous melanoma patient, regarding its risk of developing metastases. It also relates to the use of a marker gene which underwent a somatic copy number alteration (SCNA) for classifying a cutaneous melanoma patient regarding its risk of developing metastases. Furthermore, it relates to a method ex *vivo* for identifying treatment targets in a cutaneous melanoma patient. The invention also relates to a method of selecting a treatment of a cutaneous melanoma patient.

### BACKGROUND

Melanoma is the most dangerous type of skin cancer. It develops from the melanin-producing cells known as melanocytes. It typically occurs in the skin, but may rarely occur in the mouth, intestines, or eye. Melanoma is frequently referred to as malignant melanoma.

The primary cause of cutaneous melanoma is ultraviolet light (UV) exposure in those with low levels of the skin pigment melanin. The UV light may be from the sun or other sources, such as tanning devices. Those with many moles, a history of affected family members, and poor immune function are at greater risk. A number of rare genetic conditions, such as xeroderma pigmentosum, also increase the risk.

Cutaneous melanoma tends to spread early metastases (secondary tumors) via lymphatic and blood vessels and is the most common fatal skin disease, with a rapidly increasing number of new cases worldwide. Globally, in 2012, it newly occurred in 232,000 people. In 2015, 3.1 million people had active disease, which resulted in 59,800 deaths.

The treatment strategy depends primarily on the stage at which the cutaneous melanoma is detected. At an early stage, it is usually sufficient to remove the primary tumor surgically, with a sufficient safety margin, *in sano.* For more advanced cutaneous melanomas, adjuvant therapy is recommended for those with a higher tumor thickness without further lymph node involvement, as well as after removal of the lymph nodes. In later stages, when the tumor has already metastasized to the skin, lymph nodes and internal organs, the chances of recovery are slim. In these cases, a whole range of alternative therapies are used and tested, which usually only provide temporary improvement but usually have no prospect of recovery. These previously included chemotherapy with DTIC or fotemustine, surgical procedures to reduce the tumor mass, or radiotherapy. New therapeutic approaches are based on the blockade of molecular processes in the signal transduction of the cell, primarily inhibitors of BRAF^{V600E} or immune checkpoint inhibitors.

Early detection of cutaneous melanoma is therefore essential for successful treatment. In particular, a treatment option depends on the risk of developing metastases.

Current methods for predicting the risk of metastases are largely limited to histological procedures, such as determining tumor thickness or tumor stage. Other methods relate to the determination of ulceration and the mitotic rate or involve sentinel lymph node biopsy. In the latter procedure, the sentinel lymph nodes are removed for subsequent histological examination.

Molecular medical methods have been used to supplement these, such as determining the so-called 'tumor mutational burden (TMB)'. These thousands of analyses allow to describe the development of various cancers and the genomic phenotype of metastases. Yet, most importantly until now they did not allow to identify genome signatures in primary tumors that correlate with an increased or decreased risk of developing metastases.

Reschke et al., 'Identifying high-risk tumors within AJCC stage IB-III melanomas using a seven-marker immunohistochemical signature, Cancers 2021, 13, 2902, aim to validate a seven-marker immunohistochemical signature, consisting of the genes *Bax, Bcl-X, PTEN, COX-2,* β-*Catenin*, *MTAP* and *CD20.* According to the authors, the seven-marker signature is able to identify high-risk patients within stages IB-III melanoma patients that have a significantly higher risk of disease recurrence, metastasis, and death.

Nunes et al., 'Prognostic genome and transcriptome signatures in colorectal cancers', Nature 2024, Vol. 633, 137ff., disclose a number of genes which were subject to copy number variations (CNVs) or mutations, and which might be associated with metastases in colorectal cancers. These CNVs are mentioned as part of a complex scoring system. The document is, however, silent on melanomas.

Corish et al., 'The genomic landscape of 2,023 colorectal cancers', Nature 2024, Vol. 633, 127ff., examined metastatic samples of colorectal cancers and found that almost all of these samples were microsatellite stable (MSS) tumors. They analyzed the role of driver mutations in metastases and identified key genes. Also, this document is silent about melanomas.

Zeng et al., 'Biallelic loss of CDKN2A initiates melanoma invasion via BRN2 activation', Cancer Cell 2018, Vol. 34(1), describe that in a mouse model loss of the *CDKN2A* tumor suppressor is associated with melanoma metastasis. The authors speculate that CDKN2A suppresses the initiation of melanoma invasion through inhibition of BRN2.

Karras et al., 'Decoding the interplay between genetic and non-genetic drivers of metastasis', Nature 2024, Vol. 629, 543ff., is a review article which mentions *CDKN2A, MYC* und *TP35* as so-called driver genes in lung, breast, bowel and kidney cancer. It is generally referred to "genetic alterations" which could be found in such driver genes in order to promote metastases formation in such cancer types. SCNAs are mentioned in early-stage primary cancer lesions as associated with an increased risk of recurrence.

However, it has been turned out that with the current methods available, the risk of a melanoma patient to develop metastases and the decision on lymph node surgery or an adjuvant therapy with either inhibitors of BRAF^{V600E} in combination with MEK inhibitors or (combined) immune therapies with immune checkpoint inhibitors depends exclusively on three factors, melanoma thickness, the presence or absence of ulceration, and micro-metastases in the tumor draining (sentinel) lymph node. Despite uncountable attempts, all attempts failed to identify until today any reliable histological, immune or molecular marker that allows a reliable prediction of the risk of metastasis in a patient with a primary melanoma. As a result, melanoma patients are only judged on classical histological marker and often not treated adequately, e.g. subjected to adjuvant therapy without this being necessary.

### SUMMARY

In view of this, the invention is based on the objective of providing a method to avoid or at least reduce the disadvantages of the prior art. In particular, such a method is to be provided that can be used to reliably predict the risk of metastasis in cutaneous melanoma patients, starting from the primary tumor.

The object underlying the invention is met by the provision of a method ex *vivo* for classifying a cutaneous melanoma (CM) patient, regarding its risk of developing metastases, comprising the following steps:
1. providing a biological sample of the primary cutaneous melanoma,
2. analyzing the biological sample for the presence of a somatic copy number alteration (SCNA) in at least one marker gene,
3. classifying the melanoma patient as a high-risk patient if a SCNA is detected in step 2,
wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS*, *CCND3, E2F3, PPM1D, SETD81,* CD276 *(B7H3), MCL1, LAMA2, and MYC.*

The inventors have recognized a principle, namely that somatic copy number alterations (SCNAs) in a set of specific marker genes can be used to predict the likelihood of metastases forming in cutaneous melanoma. Importantly, the method according to the invention starts from the primary cutaneous melanoma and no search for metastatic tissue is required.

The method according to the invention differs significantly from those described in the prior art and currently in use. In the art it is usually searched for point mutations, single nucleotide polymorphisms or other mutations which were made responsible for metastases formation. According to the inventors' findings, however, these approaches in the art are not suitable for predicting the risk of metastasis formation in the primary melanoma. Such mutations are mostly detected with a similar frequency in melanomas that either do or do not cause metastases or typically only detected and described in the secondary tumor metastases. Here, the inventors found very specific genomic changes in the identified marker genes in the primary cutaneous melanoma that favor the later occurrence of metastases, namely the so-called 'somatic copy number alterations' (SCNAs).

The significant advantage of the method according to the invention is that it is possible to determine at a very early tumor stage using only primary cutaneous melanoma samples, whether or not the patient being examined will develop metastases. This has massive consequences for therapy. A patient with a high risk of developing metastases can undergo therapies at an early stage, what significantly increases the chances of complete cure. In contrast, a patient with a low or no risk of developing metastases, after surgical removal of the primary cutaneous melanoma, can be spared such therapies and the related toxicities.

The method according to the invention therefore allows to identify patients at risk of metastatic disease, and to select these patients for a targeted and patient-specific melanoma treatment. This was previously not possible in this form.

The invention not only helps to avoid unnecessary side effects of medical treatment, but also unnecessary treatment costs and those that would place a burden on the healthcare system without bringing any benefits for patients.

"Somatic copy number alteration" (SCNA), also known as copy number alteration (CNA) or copy number variation/variant (CNV), which terms are used herein interchangeably, refers to changes in the number of copies of specific segments of DNA within the genome that occur in somatic (non-germline) cells. Such changes can mean an amplification of the number of copies or even their complete loss. The inventors realized that, surprisingly, it is SCNAs in the marker genes that correlate with the risk of metastasis. This finding was particularly unexpected because individual genes of the OncoCycle may already been described as linked to tumorigenesis, but such are usually characterized by mutations.

In the art, however, SCNAs are described as being relevant for the transition from a benign nevus inti a melanoma; see Zeng et al., *I.c.* But, as most melanomas do not cause metastatic disease, they have not been shown to be of relevance for the development of metastases.

The method according to the invention is based on the analysis of at least one marker gene from a group of marker genes, which marker genes the inventors have named "OncoCycle".

The *GLI1* gene (Gene ID: 2735; human) encodes the zinc finger protein GLI1 also known as glioma-associated oncogene. GLI1 was originally isolated from a glioma tumor and has been found to be up regulated in many tumors including muscle, brain and skin tumors such as Basal cell carcinoma (BCC).

The *IFNE* gene (Gene ID: 338376, human) encodes interferon epsilon. It is predicted to enable cytokine activity and type I interferon receptor binding activity. Some diseases are described associated with IFNE, such as Aicardi-Goutieres Syndrome and Androgen Insensitivity Syndrome. IFNE is described as tumor suppressor that restricts ovarian cancer.

The gene *IFNGR1* (gene ID: 3459, human) encodes interferon gamma receptor 1 (IFNGR1), also known as CD119 (Cluster of Differentiation 119), which is the ligand-binding chain (alpha) of the heterodimeric gamma interferon receptor, that is found on macrophages. Mutations in the *IFNGR1* gene can lead to extreme susceptibility to My-cobacterial infections.

*JAK2* (Gene ID: 3717; human) codes for Janus kinase 2 (commonly called JAK2), which is a non-receptor tyrosine kinase. *JAK2* gene fusions with the *TEL(ETV6) (TEL-JAK2)* and *PCM1* genes have been found in patients suffering leukemia, particularly clonal eosinophilia forms of the disease. Mutations in JAK2 have been implicated in polycythemia vera, essential thrombocythemia, and myelofibrosis as well as other myeloproliferative disorders.

*ARID1B* (Gene ID: 57492; human) encodes AT-rich interactive domain-containing protein 1B, which is a component of the human SWI/SNF chromatin remodeling complex. Germline mutations in ARID1B are associated with Coffin-Siris syndrome. Somatic mutations in ARID1B are associated with several cancer subtypes, suggesting that it is a tumor suppressor gene.

The gene *CDKN2B* (Gene ID: 1030; human) encodes Cyclin-dependent kinase 4 inhibitor B also known as multiple tumor suppressor 2 (MTS-2) or p15^{INK4b}. It forms a complex with CDK4 or CDK6, and prevents the activation of the CDK kinases by cyclin D, thus the encoded protein functions as a cell growth regulator that inhibits cell cycle G1 progression. This gene lies adjacent to the tumor suppressor gene *CDKN2A* in a region that is frequently mutated, deleted, or deregulated in a wide variety of cancer.

*CDKN2C* (Gene ID: 1031; human) encodes Cyclin-dependent kinase 4 inhibitor C. It is a member of the INK4 family of cyclin-dependent kinase inhibitors. This protein has been shown to interact with CDK4 or CDK6, and prevent the activation of the CDK kinases, thus function as a cell growth regulator that controls cell cycle G1 progression. Ectopic expression of *CDKN2C* was shown to suppress the growth of human cells in a manner that appears to correlate with the presence of a wild-type RB1 function. Studies in the knockout mice suggested the roles of this gene in regulating spermatogenesis, as well as in suppressing tumorigenesis.

The gene *TP53BP1* (Gene ID: 7158; human) codes for tumor suppressor p53-binding protein 1, also known as p53-binding protein 1 or 53BP1. 53BP1 is under-expressed in most cases of triple-negative breast cancer.

*ERBB3* (Gene ID: 2065; human) encodes receptor tyrosine-protein kinase erbB-3, also known as HER3 (human epidermal growth factor receptor 3). ErbB3 is a member of the epidermal growth factor receptor (EGFR/ERBB) family of receptor tyrosine kinases. While no evidence has been found that ErbB3 overexpression, constitutive activation, or mutation alone is oncogenic, the protein as a heterodimerization partner, most critically with ErbB2, is implicated in growth, proliferation, and the promotion of invasion and metastasis. ErbB3 is also associated with targeted therapeutic resistance in numerous cancers. There are no data suggesting a potential role of *ERBB3* SNCAs in primary melanomas.

The *CDK4* gene (Gene ID: 1019; human) codes for cyclin-dependent kinase 4 (CDK4) also known as cell division protein kinase 4. It is a catalytic subunit of the protein kinase complex that is important for cell cycle G1 phase progression. Mutations in this gene as well as in its related proteins including D-type cyclins, p16(INK4a), *CDKN2A* and Rb were all found to be associated with tumorigenesis of a variety of cancers. One specific point mutation of CDK4 (R24C) was first identified in melanoma patients. This mutation was introduced also in animal models and its role as a cancer driver oncogene was studied thoroughly. Nowadays, deregulated CDK4 is considered to be a potential therapeutic target in some cancer types and various CDK4 inhibitors are being tested for cancer treatment in clinical trials. In contrast, SNCAs of the CDK4 gene were never identified in primary melanomas as a risk factor for the development melanoma metastases.

*MDM2* gene (Gene ID: 4193) encodes mouse double minute 2 homolog (Mdm2) also known as E3 ubiquitin-protein ligase Mdm2. Mdm2 is an important negative regulator of the p53 tumor suppressor. Mdm2 protein functions both as an E3 ubiquitin ligase that recognizes the N-terminal trans-activation domain (TAD) of the p53 tumor suppressor and as an inhibitor of p53 transcriptional activation. Mdm2 overexpression, in cooperation with oncogenic Ras, promotes transformation of primary rodent fibroblasts, and mdm2 expression led to tumor formation in nude mice. The human homologue of this protein was later identified and is sometimes called Hdm2. Further supporting the role of mdm2 as an oncogene, several human tumor types have been shown to have increased levels of Mdm2, including soft tissue sarcomas and osteosarcomas as well as breast tumors. SNCAs of the *MDM2* gene were identified in melanoma metastases and are thought to be specific for metastases, as SNCAs of SNCAs of the *MDM2* gene were never identified in primary melanomas as a risk factor for the development melanoma metastases.

*PIK3CA* gene (Gene ID: 5290; human) codes for phosphatidylinositol-4,5-bisphosphate 3-kinase catalytic subunit alpha. This gene has been found to be oncogenic. *PIK3CA* is the most recurrently mutated gene in breast cancer, and has been found to important in a number of cancer types.

The *HRAS* gene (Gene ID: 3265; human) encodes GTPase HRas, from "Harvey Rat sarcoma virus", also known as transforming protein p21. GTPase HRas is involved in regulating cell division in response to growth factor stimulation. HRAS has been shown to be a proto-oncogene. Mutations in the *HRAS* gene also have been associated with the progression of bladder cancer and an increased risk of tumor recurrence after treatment. Somatic mutations in the *HRAS* gene are probably involved in the development of several other types of cancer. These mutations lead to an HRAS protein that is always active and can direct cells to grow and divide without control. Recent studies suggest that HRAS mutations are common in thyroid, salivary duct carcinoma, epithelial-myoepithelial carcinoma, and kidney cancers.

*CCND3* (Gene ID: 896; human) codes for G1/S-specific cyclin-D3. The protein encoded by this gene belongs to the highly conserved cyclin family, whose members are characterized by a dramatic periodicity in protein abundance through the cell cycle. Cyclins function as regulators of CDK kinases. This cyclin forms a complex with and functions as a regulatory subunit of CDK4 or CDK6, whose activity is required for cell cycle G1/S transition. This protein has been shown to interact with and be involved in the phosphorylation of tumor suppressor protein Rb. Mutations in *CCND3* are implicated in cases of breast cancer.

The *E2F3* gene (Gene ID: 1871; human) encodes transcription factor E2F3 which belongs to the E2F family of transcription factors. The E2F family plays a crucial role in controlling the cell cycle and action of tumor suppressor proteins and is also a target of the transforming proteins of small DNA tumor viruses. This protein binds specifically to phosphorylated retinoblastoma protein pRB in a cell-cycle dependent manner.

The gene *PPM1D* (Gene ID: 8493; human) codes for protein phosphatase 1D which is a member of the PP2C family of Ser/Thr protein phosphatases. PP2C family members are known to be negative regulators of cell stress response pathways. The expression of this gene is induced in a p53-dependent manner in response to various environmental stresses. While being induced by tumor suppressor protein TP53/p53, this phosphatase negatively regulates the activity of p38 MAP kinase (MAPK/p38) through which it reduces the phosphorylation of p53, and in turn suppresses p53-mediated transcription and apoptosis. The amplification of the gene *PPM1D* has been detected in both breast cancer cell line and primary breast tumors, which suggests a role of this gene in cancer development.

The *SETDB1* gene (Gene ID: 9869; human) encodes the enzyme histone-lysine N-methyltransferase SETDB1. The SET domain is a highly conserved, approximately 150-amino acid motif implicated in the modulation of chromatin structure. Studies have suggested that the SET domain may be a signature of proteins that modulate transcriptionally active or repressed chromatin states through chromatin remodeling activities.

CD276 *(B7H3)* is a gene (Gene ID: 80381; human) that encodes CD276 molecule which belongs to the immunoglobulin superfamily and thought to participate in the regulation of T-cell-mediated immune responses. Studies show that while the transcript of this gene is ubiquitously expressed in normal tissues and solid tumors, the protein is preferentially expressed only in tumor tissues.

The *MCL1* gene (Gene ID: 4170; human) codes for induced myeloid leukemia cell differentiation protein Mcl-1. The protein encoded by this gene belongs to the Bcl-2 family. Mcl-1 works synergistically with p53 in protecting liver from injury, fibrosis and cancer. Mcl-1 has been identified as a resistance factor of cancers as it prevents apoptosis jointly with BCL-2.

*LAMA2* gene (Gene ID: 3908; human) codes for Laminin subunit alpha-2 . Laminin, an extracellular matrix protein, is a major component of the basement membrane. It is thought to mediate the attachment, migration, and organization of cells into tissues during embryonic development by interacting with other extracellular matrix components. It is composed of three subunits, alpha, beta, and gamma, which are bound to each other by disulfide bonds into a cross-shaped molecule. This gene encodes the alpha 2 chain, which constitutes one of the subunits of laminin 2 (merosin) and laminin 4 (s-merosin). Mutations in this gene have been identified as the cause of congenital merosin-deficient muscular dystrophy.

*MYC* (Gene ID: 4609; human) codes for MYC proto-oncogene, bHLH transcription factor, which is a member of the Myc family of transcription factors. This gene is a proto-oncogene and encodes a nuclear phosphoprotein that plays a role in cell cycle progression, apoptosis and cellular transformation. Amplification of this gene is frequently observed in numerous human cancers. Translocations involving this gene are associated with Burkitt lymphoma and multiple myeloma in human patients.

According to the invention "a biological sample" refers to a cellular sample of the patient comprising genetic and/or encoded protein material, which allows the analysis for genomic alterations, in particular SCNAs, in the marker genes.

"Primary cutaneous melanoma" refers to the initial tumor that arises directly from melanocytes, the pigment-producing cells in the skin, mucous membranes, or other melanocyte-containing tissues such as the eye (uveal melanoma). It represents the original site of cutaneous melanoma development before any spread to other regions of the body. The primary cutaneous melanoma is thus explicitly distinguished from the secondary or metastatic melanoma that has spread (metastasized) from the primary melanoma to distant organs or tissues.

As used herein, "high-risk patient" refers to a subject in whom the likelihood of developing metastases is significantly increased as compared to a healthy subject or a low-risk patient. In one aspect, the likelihood that distant metastases will develop within 5-10 years is at least ≥5%, preferably ≥10%, more preferably ≥20%, more preferably ≥30%, more preferably ≥50%, more preferably ≥60%. In a "high-risk patient", the 5-year survival rate is significantly lower than in a healthy subject or a low-risk patient, preferably below 50%, more preferably below 40%, more preferably below 30%, more preferably below 20%, and more preferably below 10%.

The method according to the invention is carried out "*ex vivo".* It starts from a provided biological sample of the primary cutaneous melanoma and does not require the presence of a human or animal body, and thus does not require the presence of the patient.

In a variant of the method according to the invention the two genes *MTAP* (Gene ID: 4507; human) and/or *CDKN2A* (Gene ID: 1029; human) are added to the members of marker genes of the "OncoCycle". This has the advantage that two marker genes are included which comprise a high prognostic value in indicating the risk of developing metastases in cutaneous melanoma.

Accordingly, an embodiment of the invention the SCNA is an allelic loss and/or a gene amplification.

In another embodiment of the invention the allelic loss is a homozygous (biallelic) loss.

As the inventors have found, in the case of a high risk of metastasis, both copies or alleles have been lost in a significant number of the marker genes of the OncoCycle. The loss is therefore homozygous or biallelic. According to the inventors' findings, a homozygous or biallelic loss of members of the OncoCycle appears to be a principle underlying the metastasis process. In an alternative, the SCNA is a heterozygous loss together with a loss-of-function mutation of the remained allele resulting in a biallelic functional loss.

In another embodiment of the invention, the gene amplification is a ≥3fold gene amplification.

The inventors were able to determine that at least a 3fold gene amplification had taken place in a number of marker genes of the OncoCycle in high-risk patients. According to the inventors' findings, a ≥3fold gene amplification of members of the OncoCycle appears to be a principle underlying the metastasis process. According to the invention, ≥3-fold means at least 3, 4, 5, 6, 7, 8 or more copies of the marker gene can be found.

In yet another embodiment of the invention the following SCNA is detected in the respective marker gene:

| marker gene | SCNA |
|---|---|
| *GLI1* | gene amplification |
| *IFNE* | biallelic loss |
| *IFNGR1* | biallelic loss |
| *JAK2* | biallelic loss |
| [*MTAP* | biallelic loss] |
| *ARID1B* | biallelic loss |
| [*CDKN2A* | biallelic loss] |
| *CDKN2B* | biallelic loss |
| *CDKN2C* | biallelic loss |
| *TP53BP1* | biallelic loss |
| *ERBB3* | gene amplification |
| *CDK4* | gene amplification |
| *MDM2* | gene amplification |
| *PIK3CA* | gene amplification |
| *HRAS* | gene amplification |
| *CCND3* | gene amplification |
| *E2F3* | gene amplification |
| *PPM1D* | gene amplification |
| *SETDB1* | gene amplification |
| CD276 *(B7H3)* | gene amplification |
| *MCL1* | gene amplification |
| *LAMA2* | biallelic loss |
| *MYC* | gene amplification |

With this measure, a refinement of the method according to the invention is achieved in that the specified, specific SCNAs are characteristic for the respective marker gene of the OncoCycle. The prognostic and diagnostic certainty is hereby increased even further.

In another embodiment of the invention at least 2, 3, 4, 5, 6, 7, 8, 9, 10, 11, 12, 13, 14, 15, 16, 17, 18, 19, 20 or all 21 [or, if *MTAP* and/or *CDKN2A* are included, 22, 23] marker genes of the OncoCycle are analyzed for an SCNA.

This measure has the advantage that the addition of further or several marker genes allows classification with even greater certainty and a higher probability of success. The prognostic or diagnostic certainty increases with the number of marker genes of the OncoCycles analyzed in step (2).

In still another embodiment of the invention, in step 2 at least the following marker genes of the OncoCycle are analyzed for an SCNA:
[- *GLI1, CDKN2A*; and/or]
- *GLI1, IFNE, IFNGR1,* [*CDKN2A,*] *CDK4;* and/or
- *IFNE, IFNGR1,* [*MTAP,*] *JAK2, CDK4,* [*CDKN2A*]; and/or
- *GLI1, IFNE, JAK2,* [*MTAP,*] *ARID1B,* [*CDKN2A,*] *ERBB3, CDK4, MCL1, MDM2;* and/or
- *GLI1, IFNE, IFNGR1,* [*MTAP,*] *ARID1B,* [*CDKN2A,*] *ERBB3, CDK4, MCL1, MDM2, ARID1B, SETD81, HRAS1, E2F3,* CD276; and/or
- *GLI1, IFNE, IFNGR1, JAK2,* [*MTAP,*] *ARID1B,* [*CDKN2A*]*, ERBB3, CDK4, MCL1, MDM2, SETD81, ARID1B, HARAS1, E2F3,* CD276; and/or
- *GLI1, IFNE, IFNGR1, JAK2,* [*MTAP,*] [*CDKN2A,*] *CDKN2B, CDKN2C, TP53BP1, ARID1B, ERBB3, CDK4, MDM2, PIK3CA, HRAS, CCND3, E2F3, PPM1D, SETD81,* CD276 *(B7H3), MCL1, LAMA2, MYC.*

According to the findings of the inventors, the specified combinations of marker genes of the OncoCycle are those that are frequently found altered by SCNAs in high-risk patients. In this embodiment of the method according to the invention, the prognostic significance is therefore further improved.

The square brackets indicate that *MTAP* and *CDKN2A* can optionally be included in the OncoCycle, but it is not necessary.

In another embodiment of the method according to the invention the following additional step is carried out:
2'. analyzing the biological sample for determining the tumor thickness of the primary tumor.

With this measure, the method according to the invention is modified by an additional step, by which customary methods for determining the risk of metastasis are included. The inventors were able to determine that the inclusion of determination of thickness of the primary cutaneous melanoma again increases the prognostic certainty. According to the invention, the tumor thickness can be determined by methods known to those skilled in the art. These include, among others, the so-called tumor thickness according to Breslow or Breslow level. This is a histological classification used to assess the stages of a cutaneous melanoma and the prognosis derived from it. In this process, the absolute thickness of the tumor tissue is measured from the stratum granulosum of the epidermis to the deepest detectable tumor tissue in surgically removed cancer tissue. The tumor thickness is then given in millimeters. Another histological method is the so-called Clark level. In this method, the deepest detectable tumor tissue is determined from the skin surface in the surgically removed cancer tissue and its infiltration into the surrounding healthy skin areas during a microscopic examination. The result of the tumor thickness determination is combined with the analysis of SCNAs of members of the OncoCycle in the biological sample, which allows an even better classification of melanoma patients.

In yet another embodiment of the method according to the invention the following additional step is carried out:
2". analyzing the biological sample for determining the tumor stage.

This additional step also implements a conventional method for determining the risk of metastasis in the method according to the invention. The latter thus provides even better information on the probability of metastasis. For example, the tumor stage can be determined according to the TNM classification developed by the American Joint Committee on Cancer (AJCC, Edition 8). In stage I, melanomas are confined to the skin and are thin (up to 2 mm), without ulceration or with minimal ulceration. In these early stages, the risk of metastasis is relatively low. In stage II, melanomas are thicker (> 2 mm) and/or ulcerated, but there is no evidence of lymph node involvement or distant metastases. In stage III, melanoma cells have spread to nearby regional lymph nodes or there are satellite metastases (tumor cells near the original melanoma site but not in the lymph nodes). The risk of systemic spread is high. Stage IV describes the presence of distant metastases in more distant organs, such as the lungs, liver, brain, bones or other internal organs. This is the most advanced stage of melanoma and indicates systemic disease. The result of the tumor stage determination is combined with the analysis of SCNAs of members of the OncoCycle in the biological sample, which allows an even better classification of melanoma patients.

In still another embodiment of the invention the following additional step is carried out:
2‴. analyzing the biological sample for determining the tumor mutational burden (TMB).

This additional step also implements an established technique to determine the risk of metastasis. The method according to the invention thus becomes even more reliable. Tumor mutational burden (abbreviated as TMB) is a genetic characteristic of tumorous tissue, which is defined as the number of non-inherited mutations per million bases (Mb) of investigated genomic sequence. TMB is also strongly predictive of overall as well as disease-specific survival, independently of cancer type, stage or grade. Typically, whole genome sequencing, whole exome sequencing, and panel based approaches are used to calculate TMB. The result of the TMB determination is combined with the analysis of SCNAs of members of the OncoCycle in the biological sample, which allows an even better classification of melanoma patients.

In yet a further embodiment in the method according to the invention the following additional step is carried out:
2‴ʺ. analyzing the biological sample for determining the copy number variant (CNV) burden.

This additional step also provides additional prognostic certainty, because determining the copy number variant (CNV) burden also allows the risk of metastasis to be determined. Copy number variant (CNV) burden refers to the total extent of DNA that is affected by copy number variations (CNVs) within a genome. In the context of cutaneous melanomas, CNV burden has been linked by the inventors to the progression of primary melanomas into metastatic disease. Melanomas are characterized by especially extensive genomic alterations. Key genes involved in the transition of benign nevi into primary melanomas, cell cycle regulation, and apoptosis may be impacted by CNVs, leading to dysregulation of pathways critical for cell growth and survival. CNV burden is typically determined using high-throughput genomic techniques such as Array Comparative Genomic Hybridization (aCGH), Single Nucleotide Polymorphism (SNP) Arrays, Next-Generation Sequencing (NGS), or Whole Genome Sequencing (WGS). Once CNVs are identified, CNV burden is typically calculated by summing the number and/or size of all CNVs across the genome. The result of the CNV burden determination is combined with the analysis of SCNAs of members of the OncoCycle in the biological sample, which allows an even better classification of melanoma patients.

In an embodiment of the method according to the invention the following additional step is carried out:
3'. prognosing a reduced progression-free survival (PFS) for the patient if a SCNA is detected in step 2.

This additional step makes it possible not only to provide information about the risk of metastasis but also to make a prognosis about the probability of survival of the affected patient. Both patient and physician thus receive further important information that can have a beneficial effect on the treatment approach, life planning and mental state of the patient. Progression-Free Survival (PFS) is a clinical endpoint used in cancer studies and trials to measure the length of time during and after treatment that a patient lives with the disease without the cancer worsening. It is a key metric to assess the efficacy of a treatment in slowing or halting disease progression. PFS is the time from the start of treatment (or the time of randomization in clinical trials) to the point at which the cancer progresses or the patient dies from any cause, whichever occurs first. In other words, PFS measures how long a patient can survive without the disease worsening (progressing). PFS is determined by regularly scheduled clinical evaluations. These assessments typically include imaging tests (e.g., ultrasound scans, CT scans, MRI, PET scans) to track tumor size and spread, physical exams to assess clinical symptoms, and biomarkers or laboratory tests that might signal disease progression. PFS is often displayed using Kaplan-Meier survival curves in clinical trials, showing the proportion of patients who remain progression-free over time.

In still another embodiment of the invention, in step (2) of the method the biological sample is analyzed via DNA analysis, preferably via genome/exome/panel sequencing.

In yet another embodiment of the invention in step (2) of the method the biological sample is analyzed via RNA analysis, preferably via RNA sequencing and/or single cell transcriptomics, and/or spatial transcriptomics.

These measures use methods that are well established in molecular biology and molecular diagnostic laboratories and with which any genomic alterations in the marker genes can be reliably detected. This provides experts with methodologies that enable the procedure to be carried out in everyday laboratory work.

In a further embodiment of the method according to the invention in step (2) the biological sample is analyzed via Fluorescence in situ hybridization (FISH).

Fluorescence in situ hybridization (FISH) is a molecular biology technique used to detect and localize the presence or absence of specific DNA sequences on chromosomes. It is particularly useful for identifying genomic alterations, such as copy number variations (CNVs), gene rearrangements, translocations, deletions, and amplifications in cells and tissues. FISH uses fluorescently labeled DNA probes that bind or hybridize to complementary DNA sequences within a sample (usually metaphase chromosomes or interphase nuclei). When the sample is viewed under a fluorescence microscope, the fluorescent probes highlight specific regions of the genome where the DNA sequence of interest is located. This allows to visualize and quantify specific genetic features at the molecular level. In cases of gene amplification, FISH can reveal increased copies of a gene by showing more fluorescent signals for the targeted region compared to normal cells. Conversely, if a region of DNA is deleted, fewer fluorescent signals will be detected than in normal cells, indicating a loss of that genomic region.

In an embodiment of the method according to the invention in step (2) the biological sample is analyzed via protein analysis, preferably via Western blot, and/or immunohistochemistry, and/or immunofluorescence.

Protein analysis is a crucial tool for determining genomic alterations, such as copy number variations, on the protein level. When a genomic alteration, such as a CNV, occurs in a marker gene, the amount of protein produced by that gene may change. Protein analysis allows to measure these changes and assess the impact of genomic variations on protein levels. For example, gene amplifications typically lead to an increase in the production of the corresponding protein, while deletions can reduce or eliminate protein production. Key protein analyses methods include Western blotting (immunoblotting), enzyme-linked immunosorbent assay (ELISA), immunofluorescence, mass spectrometry, immunohistochemistry (IHC), flow cytometry, proteomics, etc.

Another subject-matter of the invention relates to the use of a marker gene which underwent a somatic copy number alteration (SCNA) for classifying a cutaneous melanoma (CM) patient, regarding its risk of developing metastases, wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS, CCND3, E2F3, PPM1D, SETDB1,* CD276 *(B7H3), MCL1, LAMA2, and MYC.*

The embodiments, features, characteristics, advantages and aspects described in the context of the classification method according to the invention also apply, in an identical or corresponding manner, to the use according to the invention.

Another subject-matter of the invention relates to a method ex *vivo* for identifying treatment targets in a cutaneous melanoma (CM) patient, comprising the following steps:
1. providing a biological sample of the primary cutaneous melanoma,
2. analyzing the biological sample for the presence of a somatic copy number alteration (SCNA) in at least one marker gene,
3. identifying a marker gene as a treatment target if a SCNA is detected in step 2,
wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS, CCND3, E2F3, PPM1D, SETD81,* CD276 *(B7H3), MCL1, LAMA2, and MYC.*

This method makes it possible to identify the marker gene as a treatment target and to individually tailor the therapy that should be administered to the patient. The treatment can then be designed to influence and, if necessary, correct the activity of the genetically modified marker gene product or treatment target. For example, if an amplified or lost marker gene leads to an acceleration of the cell cycle, the treating therapist will consider the use of a cell cycle inhibitor. If the amplification or loss leads to an increase in cell proliferation, a proliferation inhibitor will be selected as a treatment option. A specific inhibitor against the individual marker gene product may also be considered.

The embodiments, features, characteristics, advantages and aspects described in the context of the classification method according to the invention also apply, in an identical or corresponding manner, to the target identification method.

Another subject-matter of the invention relates to a method of treatment of a cutaneous melanoma (CM) patient, comprising the application of adjuvant or neo-adjuvant therapy, if, after the patient was subjected to the target identification method, a marker gene which underwent SCNA was detected in step 3.

Such method can also be understood as a method of selecting a treatment of a cutaneous melanoma (CM) patient, comprising the selection of adjuvant or neo-adjuvant therapy, if, after the patient was subjected to the target identification method, a marker gene which underwent SCNA was detected in step 3. "Selecting" in this context means that a decision making takes place in such a way that it is determined that the patient should receive adjuvant or neo-adjuvant therapy, but without any treatment being given. Such method is, therefore, carried out ex *vivo.*

Both adjuvant therapy and neo-adjuvant therapy are treatment strategies used in cancer care, including cutaneous melanoma, to reduce the risk of metastasis formation, which is the spread of cancer from the primary site to other parts of the body. The decision to use these therapies, and how they are administered, largely depends on the risk of developing metastases. Adjuvant therapy is administered after the primary treatment (usually surgery) to reduce the risk of metastasis by eliminating microscopic or controlling cancer cells that might have spread but are undetectable with current diagnostic tools. The decision to use adjuvant therapy depends on the risk of residual cancer cells forming metastases. In high-risk patients, even if the primary tumor is surgically removed, there may still be undetectable micrometastases (small clusters of cancer cells that have spread) in the body. Adjuvant therapy, such as immunotherapy or targeted therapy, is given to eliminate or control these cells, thereby lowering the risk of metastasis and cancer recurrence. In low-risk patients surgery alone may be sufficient to remove the tumor, and adjuvant therapy may not be necessary. In these cases, patients are often monitored closely with follow-up visits, but additional therapy is generally not administered, as the likelihood of microscopic metastases is low, and the risks of side effects from therapy may outweigh the benefits.

In an embodiment of the treatment and/or selection method the therapy is related to the marker gene.

This measure ensures that the treatment is precisely tailored to the genetic defect and thus to the diseased patient. This drastically increases the probability of a successful treatment. In contrast, therapies that are not as effective are excluded and unnecessary side effects are avoided. This also helps to reduce treatment costs and relieve the burden on the healthcare system. "Related to the marker gene" in this context means that the therapy aims to address and, if necessary, correct the specific and concretely amplified or lost marker gene or marker gene product.

In yet another embodiment of the treatment and/or selection method, the therapy is selected according to the genetically modified marker gene as follows:

| Marker gene | Therapy |
|---|---|
| *GLI1* | GLI1 and BET inhibitors, Hedgehog inhibitors, arsenic trioxide |
| *IFNE* | Cytokine therapy and/or cell cycle inhibitors, immune therapy secondary |
| *IFNGR1* | Cell cycle inhibitors, immune therapy secondary |
| *JAK2* | Cell cycle inhibitors, immune therapy secondary |
| *ARID1B* | HDAC inhibitors, cell cycle inhibitors, immune checkpoint blockade, inhibitors of mTOR, EZH2, histone deacetylases, ATR and/or PARP |
| *CDKN2B* | Cell cycle inhibitors |
| *CDKN2C* | Cell cycle inhibitors |
| *TP53BP1* | Cell cycle inhibitors and/or p53 activators |
| *ERBB3* | ERBB family inhibitors |
| *CDK4* | Cell cycle inhibitors |
| *MDM2* | Cell cycle inhibitors |
| *PIK3CA* | PI3K pathway inhibitors |
| *HRAS* | RAS inhibitors and/or RAS pathway inhibitors |
| *CCND3* | Cell cycle inhibitors |
| *E2F3* | Cell cycle inhibitors |
| *PPM1D* | Cell cycle inhibitors and/or PPM1D inhibitors |
| *SETDB1* | SETDB1 inhibitors, immune checkpoint blockade and/or inhibitors of mTOR, EZH2, histone deacetylases, ATR and/or PARP |
| *CD276 (B7H3)* | Anti-CD276 |
| *MCL1* | MCL1 inhibitors and/or apoptosis promoting drugs |
| *LAMA2* | |
| *MYC* | Cell cycle inhibitors |

This measure already provides the appropriate therapy or the appropriate therapeutic agent depending on the genetically modified marker gene. Treatment errors are largely excluded as a result.

"GLI1 inhibitors" are compounds that specifically inhibit the activity of GLI1, a transcription factor involved in the Hedgehog (Hh) signaling pathway. GLI1 inhibitors work by blocking the activity of GLI1 either directly (by binding to the GLI1 protein) or indirectly (by inhibiting upstream components of the Hh pathway that activate GLI1, such as Smoothened (SMO)). This results in decreased transcription of GLI1 target genes, which are involved in promoting cell proliferation and survival, particularly in cancers. Examples of GLI inhibitors include GANT61, which is a small-molecule that blocks the DNA-binding activity of GLI1, preventing it from activating target genes. GANT61 has shown preclinical efficacy in various cancers by inducing cell death and reducing tumor growth. Another GLI1 inhibitor is arsenic trioxide (ATO). Although primarily used in treating acute promyelocytic leukemia (APL), ATO has been found to inhibit GLI1 by promoting its degradation, and it is being investigated for its potential in treating cancers driven by Hedgehog pathway dysregulation. The GLI signaling can also be silenced with azole derivatives.

"BET inhibitors" target bromodomain and extra-terminal (BET) proteins, which are epigenetic regulators that control gene expression by recognizing and binding to acetylated lysine residues on histone proteins. BET inhibitors block the interaction between BET proteins and acetylated histones, preventing BET proteins from recruiting the transcriptional machinery necessary for gene expression. This inhibition reduces the transcription of oncogenes, such as *MYC*, and other genes involved in cancer cell proliferation and survival. Examples include JQ1 that selectively binds to the bromodomains of BET proteins, disrupting their interaction with chromatin. JQ1 has shown promise in preclinical cancer models and some solid tumors. I-BET762 (molibresib) is another small-molecule BET inhibitor that has shown antitumor activity in both preclinical and clinical trials. CPI-0610, another BET inhibitor in clinical trials, which has been evaluated in various cancers.

"Hedgehog inhibitors" target components of the Hedgehog (Hh) signaling pathway, which is essential for embryonic development and tissue regeneration but is often aberrantly activated in cancers, leading to uncontrolled cell growth. The core of the Hedgehog pathway involves the Hedgehog ligands (Sonic, Indian, and Desert Hedgehog), Patched (PTCH), Smoothened (SMO), and the GLI transcription factors. Hh signaling inhibitors typically block one of the key steps in the Hh pathway. The most common target is SMO, a protein that propagates Hh signaling downstream to activate GLI transcription factors. Inhibiting SMO prevents the activation of the GLI family of transcription factors, thereby blocking the transcription of Hh target genes involved in proliferation and survival. Hh signalling inhibitors include Vismodegib (Erivedge), which is approved for the treatment of advanced basal cell carcinoma (BCC), particularly for patients with locally advanced or metastatic disease who are not suitable for surgery or radiation. Vismodegib blocks the Hedgehog pathway by binding to SMO, thus inhibiting GLI activation. Sonidegib (Odomzo) is another SMO inhibitor approved for the treatment of locally advanced basal cell carcinoma that has recurred after surgery or radiation therapy. Sonidegib, like Vismodegib, works by inhibiting SMO and thereby preventing Hedgehog pathway activation. Glasdegib (Daurismo), still another SMO inhibitor, is approved for use in combination with low-dose cytarabine for the treatment of newly diagnosed acute myeloid leukemia (AML) in patients who are not candidates for intensive chemotherapy. Further Hh pathway inhibitors are Itraconazole and Saridegib (IPI-926).

"Cytokine therapy" is a form of immunotherapy that involves the use of cytokines to boost the body's immune defenses, promote the killing of cancer cells, or modulate inflammatory responses. These cytokines include interferons (IFNs), in particular type I interferons like IFN-α or IFN-ε. IFN-ε stimulates the activation of innate immune cells such as macrophages, dendritic cells, and epithelial cells at mucosal surfaces.

"Cell cycle inhibitors" are compounds or drugs that block or interfere with specific phases of the cell cycle, the process by which cells grow and divide. The cell cycle consists of several key phases: G1 (growth phase), S (DNA synthesis), G2 (preparation for mitosis), and M (mitosis or cell division). Cell cycle inhibitors target proteins involved in regulating these checkpoints, particularly proteins such as cyclins, cyclin-dependent kinases (CDKs), and their inhibitors. These drugs are used in cancer therapy to stop the proliferation of cancer cells by halting their progression through the cell cycle, thus preventing tumor growth and spread. CDKs are enzymes that, when bound to cyclins, phosphorylate specific proteins required for cell cycle progression. By inhibiting CDKs, cell cycle inhibitors block the transition between cell cycle phases, particularly the transition from the G1 phase to the S phase or from the G2 phase to mitosis. Certain proteins act as checkpoints during the cell cycle, ensuring that conditions are favorable for division and that DNA is accurately replicated. Cell cycle inhibitors disrupt these checkpoints, leading to cell cycle arrest. Examples of cell cycle inhibitors include specific CDK4/6 Inhibitors, such as Palbociclib (Ibrance), Ribociclib (Kisqali), and Abemaciclib (Verzenio). Further, there are now specific CDK2 inhibitors, such as Milciclib (PHA-848125). Aurora kinase inhibitors include Alisertib (MLN8237), and Barasertib (AZD1152). The HK1/CHK2 inhibitors include Prexasertib (LY2606368). The so-called Wee1 Inhibitors include Adavosertib (AZD1775). Polo-like kinase (PLK) inhibitors encompass volasertib (Bl 6727). Further cell-cycle inhibitors are well known to the skilled artisan.

"PRMT5 inhibitors" are compounds that inhibit Protein Arginine Methyltransferase 5 (PRMT5), an enzyme that catalyzes the methylation of arginine residues on histones and other proteins. PRMT5 plays an important role in regulating gene expression, splicing, and cell signaling by adding methyl groups to target proteins. Overexpression or hyperactivity of PRMT5 has been linked to various cancers. PRMT5 inhibitors work by blocking the methyltransferase activity of PRMT5, which results in altered gene expression and disruption of processes essential for cancer cell survival and proliferation. Examples of PRMT5 inhibitors include GSK3326595 (EPZ015938), JNJ-64619178, PF-06939999, and AMG 193.

"p53 activators" are compounds or drugs designed to restore or enhance the function of the p53 protein, a key tumor suppressor involved in regulating cell cycle arrest, DNA repair, apoptosis, and senescence in response to cellular stress or DNA damage. p53 activators aim to reactivate mutated p53 and/or enhance wild-type p53 activity. Some compounds bind to mutated p53 and restore its normal conformation, allowing it to regain its transcriptional activity and promote apoptosis in cancer cells. Other p53 activators inhibit proteins like MDM2 (murine double minute 2) and MDMX that normally bind to p53 and target it for degradation. By blocking MDM2 or MDMX, these drugs stabilize p53 levels and enhance its tumor-suppressing functions. Some activators modify the post-translational modifications of p53, such as phosphorylation or acetylation, enhancing its stability and activity. Examples of p53 inhibitors include the MDM3 inhibitors Nutlin-3, RG7112, and Idasanutlin (RG7388). MK-8242 is another MDM2 inhibitor that has shown promise in clinical trials for AML. It works by preventing the degradation of wild-type p53, thereby allowing it to activate its apoptotic functions in cancer cells. MDM2 is a negative regulator of p53, promoting its degradation through the ubiquitin-proteasome pathway. By inhibiting MDM2, these compounds prevent p53 degradation, allowing it to accumulate and become active in triggering tumor-suppressive responses. APR-246 (Eprenetapopt) works by covalently modifying cysteine residues in the p53 protein, reactivating its tumor-suppressive functions. RITA (Reactivation of p53 and Induction of Tumor cell Apoptosis) is a small molecule that binds directly to p53, preventing its interaction with MDM2 and allowing p53 to accumulate and induce apoptosis in cancer cells. CP-31398 is a p53-stabilizing compound that restores the normal folding and function of mutant p53, allowing it to bind DNA and activate p53 target genes.

"ERBB family inhibitors" are drugs or compounds that target and inhibit the activity of members of the ERBB family of receptor tyrosine kinases. ERBB family inhibitors block the kinase activity of these receptors, preventing the phosphorylation of downstream signaling molecules that promote cell proliferation and survival. Some ERBB inhibitors work by blocking the binding of growth factors (ligands) to the receptors or preventing the receptors from forming dimers, which is necessary for activation. Certain inhibitors may also promote the degradation of ERBB receptors, further reducing their activity. EGFR (ERBB1) inhibitors include Gefitinib (Iressa), Erlotinib (Tarceva), Afatinib (Gilotrif), Osimertinib (Tagrisso), and Cetuximab (Erbitux). Examples of HER2 (ERBB2) inhibitors are Trastuzumab (Herceptin), Pertuzumab (Perjeta), Lapatinib (Tykerb), Neratinib (Nerlynx), Tucatinib (Tukysa). There are described HER3 (ERBB3) inhibitors, such as Patritumab (U3-1287), and Pan-ERBB Inhibitors (multiple ERBB family targets), including fatinib (Gilotrif) and Dacomitinib (Vizimpro).

"PI3K pathway inhibitors" are compounds that target and inhibit the activity of proteins in the phosphoinositide 3-kinase (PI3K) pathway, a critical signaling pathway involved in regulating cell growth, proliferation, survival, metabolism, and angiogenesis. The PI3K/AKT/mTOR pathway is frequently dysregulated in many cancers due to mutations, amplifications, or aberrant activation of key components such as PI3K, AKT, or mTOR. This dysregulation leads to uncontrolled cell proliferation and survival, making the pathway a significant target for cancer therapies. Examples of PI3K pathway inhibitors comprise Alpelisib (Piqray), Idelalisib (Zydelig), Copanlisib (Aliqopa), and Duvelisib (Copiktra), all of which target a subunit of PI3K. Capivasertib (AZD5363) and Ipatasertib inhibit the AKT component. mTOR is inhibited by Everolimus (Afinitor) and Temsirolimus (Torisel). Dual PI3K/mTOR inhibitors include Apitolisib (GDC-0980) and Voxtalisib (SAR245409).

"RAS inhibitors" are drugs or compounds that specifically target the activity of the RAS proteins, which are key molecular switches that regulate cell proliferation, differentiation, and survival. The RAS family of proteins (which includes HRAS, KRAS, and NRAS) are small GTPases that function in the RAS/RAF/MEK/ERK signaling pathway. "RAS pathway inhibitors" encompass a broader category of drugs that target not only RAS proteins directly but also various downstream components of the RAS signaling pathway, including RAF, MEK, and ERK, which are activated by RAS. Targeting the RAS protein directly to prevent its activation or interaction with downstream effectors (e.g., blocking the binding of RAS to GTP or interfering with its membrane localization). Targeting downstream components of the RAS signaling pathway (e.g., RAF, MEK, ERK) aim to disrupt the aberrant signaling caused by RAS. Direct RAS inhibitors are, e.g., Sotorasib (Lumakras) and Adagrasib (Krazati). Examples of RAS Pathway Inhibitors include RAF inhibitors such as Vemurafenib (Zelboraf) and Dabrafenib (Tafinlar), MEK inhibitors such as Trametinib (Mekinist) and Cobimetinib (Cotellic), and ERK inhibitors such as Ulixertinib (BVD-523) and LY3214996.

"PPM1D inhibitors" are compounds that target and inhibit the activity of PPM1D (Protein Phosphatase, Mg²⁺/Mn²⁺ Dependent 1D), also known as WIP1 (Wild-type p53-induced phosphatase 1). PPM1D is a serine/threonine phosphatase that plays a crucial role in regulating the DNA damage response and maintaining cellular homeostasis by deactivating important proteins involved in cell cycle regulation and stress responses. PPM1D inhibitors work by blocking the phosphatase activity of PPM1D, preventing it from deactivating critical proteins involved in the DNA damage response. This inhibition leads to the continued activation of tumor suppressors like p53 and DNA repair proteins like ATM/ATR, thereby promoting apoptosis or senescence in cancer cells with high DNA damage. Specific examples of PPM1D inhibitors include GSK2830371 and JMS-053.

"SETDB1 inhibitors" are compounds that target and inhibit the activity of SETDB1 (SET domain bifurcated 1), an important histone methyltransferase enzyme responsible for adding methyl groups to histone H3 at lysine 9 (H3K9). This modification, known as H3K9 trimethylation (H3K9me3), is associated with transcriptional repression and the silencing of gene expression, particularly in heterochromatin regions. SETDB1 plays a critical role in regulating gene silencing, maintaining genomic stability, and controlling the expression of repetitive elements in the genome. SETDB1 inhibitors block the methyltransferase activity of SETDB1, preventing the methylation of H3K9. This results in reduced levels of transcriptional silencing and may reactivate tumor suppressor genes or other genes that are epigenetically silenced in cancer cells. Inhibiting SETDB1 can also lead to destabilization of repetitive elements and activation of an anti-tumor immune response. Examples of SETDB1 inhibitors include A-366, UNC0638 and BIX-01294.

"Anti-CD276" (Anti-B7-H3) refers to therapeutic agents, typically monoclonal antibodies, that target CD276, also known as B7-H3, a member of the B7 family of immune checkpoint proteins. CD276 is an immune regulatory molecule that is overex-pressed on various tumor cells and in the tumor microenvironment, but it has limited expression in most normal tissues. Anti-CD276 antibodies can bind to CD276 on tumor cells, marking them a target for destruction by the immune system. Some anti-CD276 therapies are developed as antibody-drug conjugates (ADCs), where the anti-CD276 antibody is linked to a cytotoxic agent that is delivered directly to the tumor cells expressing CD276. Examples of anti-CD276 compounds include Enoblituzumab (MGA271), Omburtamab (8H9), DS-7300, MGC018, and 177Lu-DOTA-Omburtamab.

"MCL1 inhibitors" are drugs or compounds that specifically target and inhibit the activity of MCL1 (Myeloid Cell Leukemia 1), an anti-apoptotic member of the BCL-2 family of proteins. MCL1 plays a critical role in regulating apoptosis (programmed cell death) by preventing the activation of pro-apoptotic proteins like BAX and BAK, which are essential for the mitochondrial pathway of apoptosis. MCL1 is frequently overex-pressed in various cancers. By inhibiting MCL1, these drugs aim to restore the apoptosis process in cancer cells, leading to cancer cell death. Examples of MCL1 inhibitors include S63845, AMG 176, AMG 397, and AZD5991.

There are other "apoptosis promoting drugs" designed to restore or enhance the process of apoptosis, such as BCL-2 family inhibitors (e.g. Venetoclax (Venclexta), Navitoclax (ABT-263), Obatoclax (GX15-070)), or SMAC mimetics (e.g. LCL161, Birinapant, Debio 1143 (AT-406)), or TRAIL agonists (e.g. Dulanermin (rhTRAIL), Tigatuzumab (CS-1008)), or protease inhibitors (e.g. Bortezomib (Velcade), Carfilzomib (Kyprolis), or HDAC inhibitors (e.g. Vorinostat (Zolinza), Romidepsin (Istodax).

It is understood that the features mentioned above and those yet to be explained below can be used not only in the particular combination given, but also in other combinations or in isolation, without departing from the scope of the present invention.

The invention is now further described and explained in further detail by referring to the following non-limiting examples and figures.

### BRIEF DESCRIPTION OF THE FIGURES

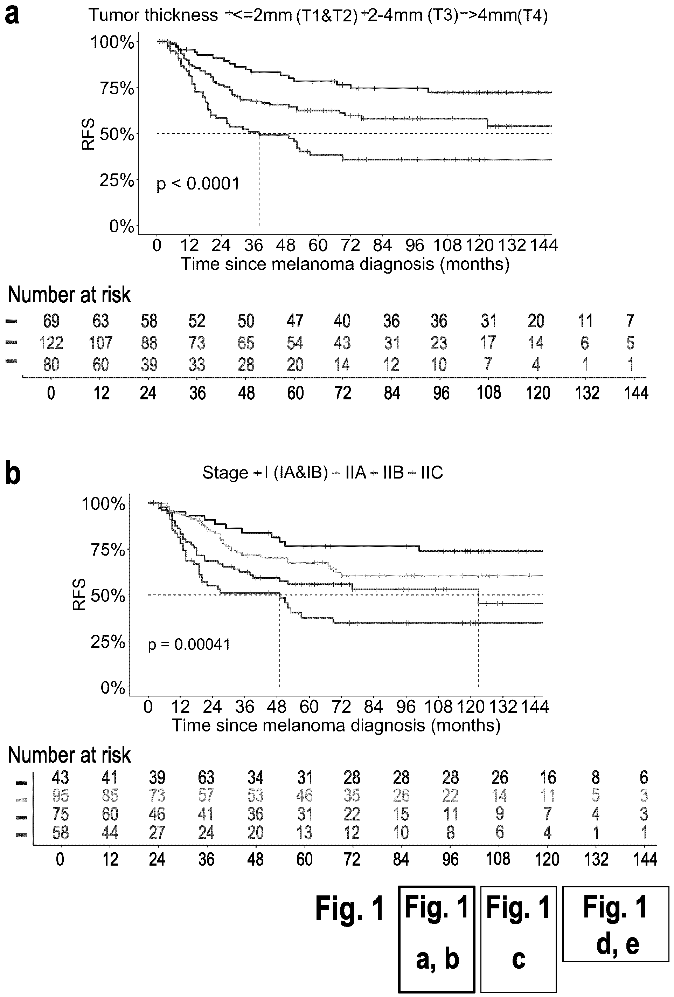

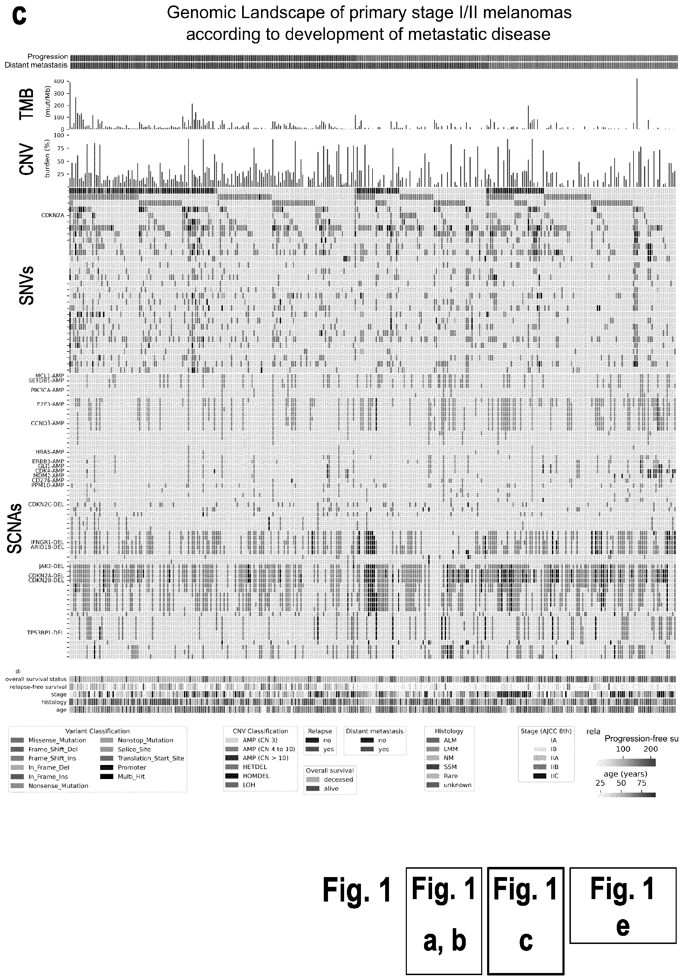

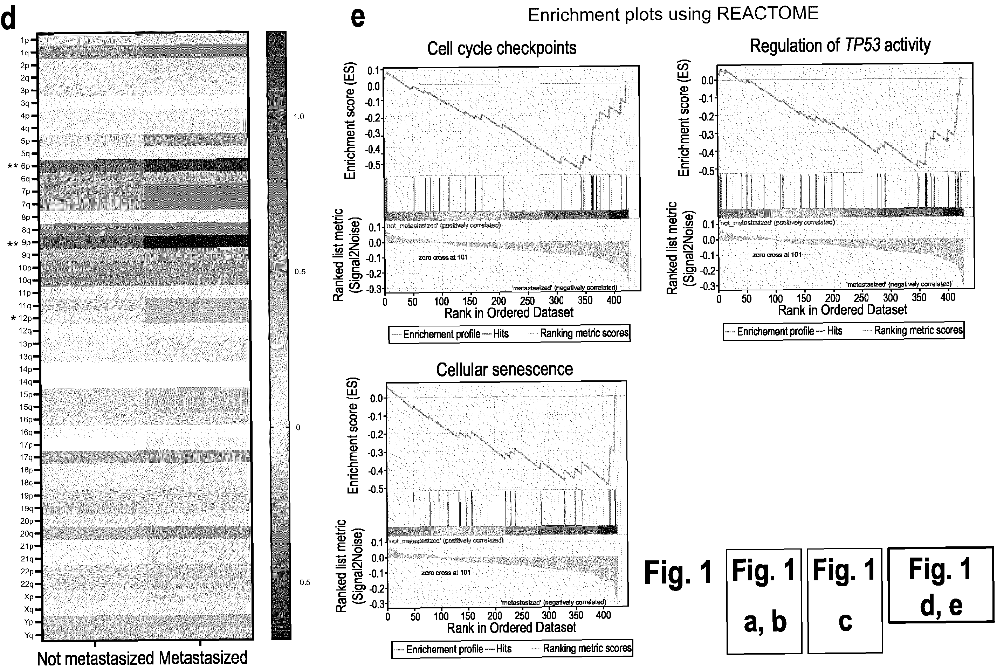

- Fig. 1:: Kaplan-Meier curves illustrating the 150-month progression-free survival (PFS) of the 271 patients, stratified according to (a) tumor thickness and (b) stage AJCC edition 8., log rank compares the survival curves for all their follow up time; (c) Oncoplot displaying genomic alterations in the 271 primary melanomas, with patients dichotomized based on the development of metastatic disease (progression). The upper section shows the genes frequently affected by oncogenic single nucleotide variants (SNVs), arranged in order of their frequency. The lower section includes the 22/23 genes affected by somatic copy number alterations (SCNAs) significantly associated with worse PFS in univariate Cox regression arranged in order of their genomic position; (d) Heatmap illustrating the normalized mean large chromosome arm gains and losses in the non-metastasized and metastasized patients. Large deletions and amplifications are defined as changes involving the entire/partial chromosome, or a whole/partial arm. Depicted are some genes identified also through GISTIC analysis located in this chromosomal region; (e) Gene Set Enrichment Analysis (GSEA) showing the enrichment of gene sets in the metastasized cohort, analyzed using the REACTOME database.

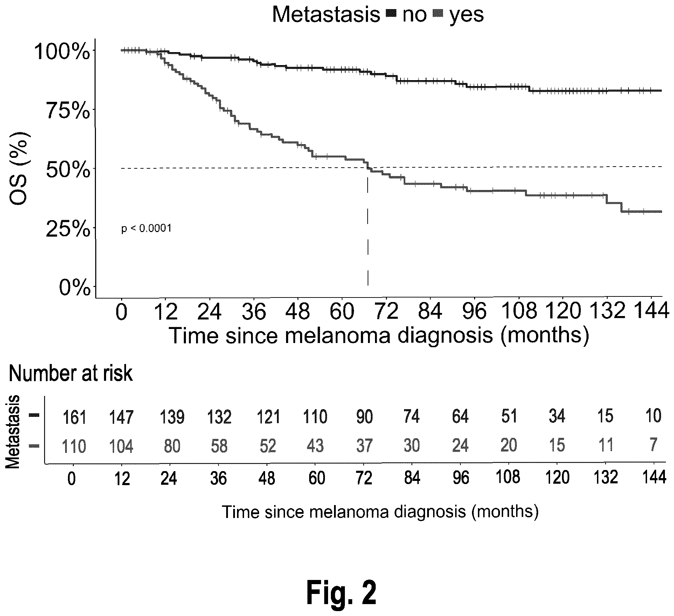

- Fig. 2:: Kaplan Meier curve for overall survival of the entire population (n=271) stage I/II melanoma patients. Patients with metastasis had a shorter overall survival.

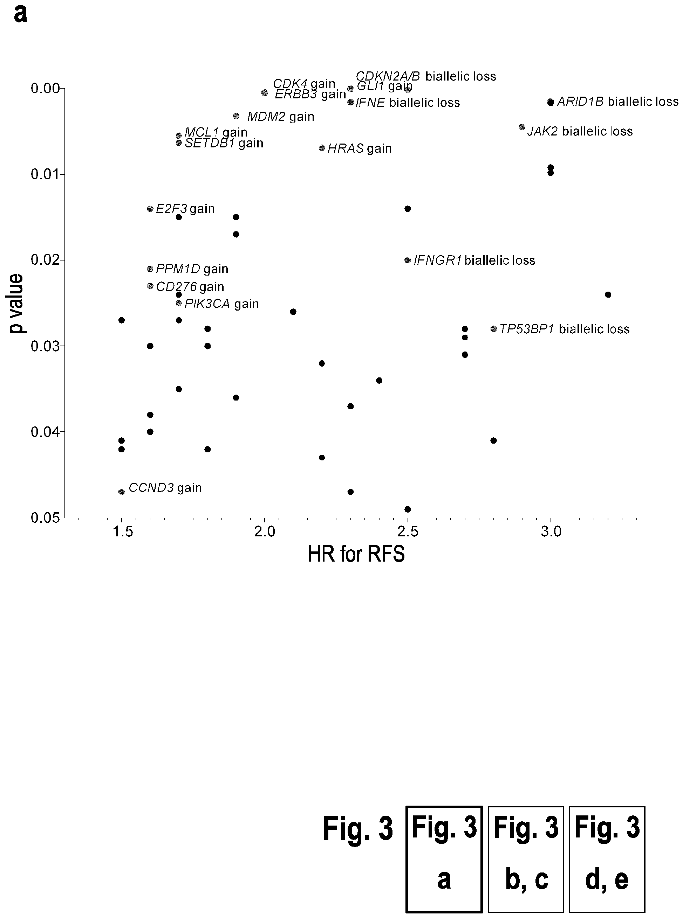

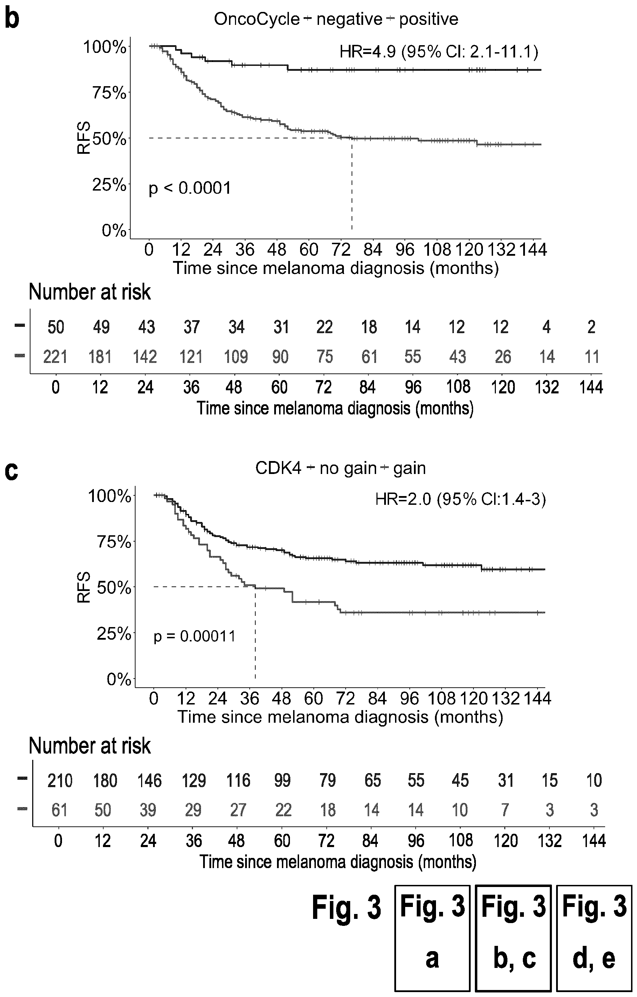

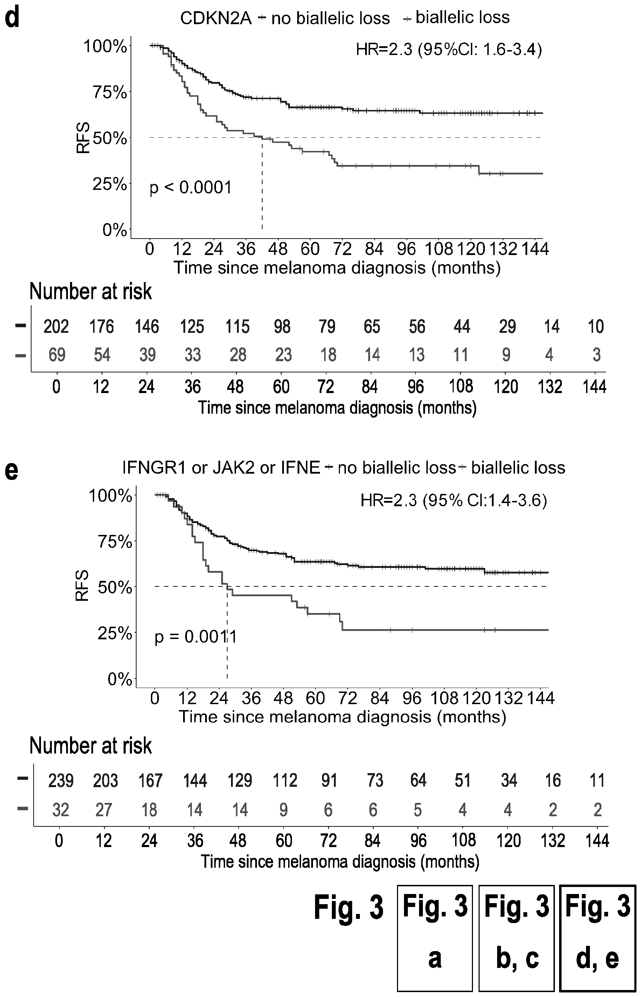

- Fig. 3:: (a) Plot showing the hazard ratio (HR) and the p value for genomic alterations of the 271 patients associated with significantly shortened PFS; Kaplan Meier curves illustrating the 150-month PFS of the 271 patients according to (b) OncoCycle status; (c) CDK4 gain; (d) CDKN2A biallelic loss; (e) defects in type I and II IFNs signaling, defined as biallelic loss in JAK2, IFNGR1 and type I interferons like IFNE. The log rank compares the survival curves for all their follow up time; the Cox regression is for the 150-month PFS.

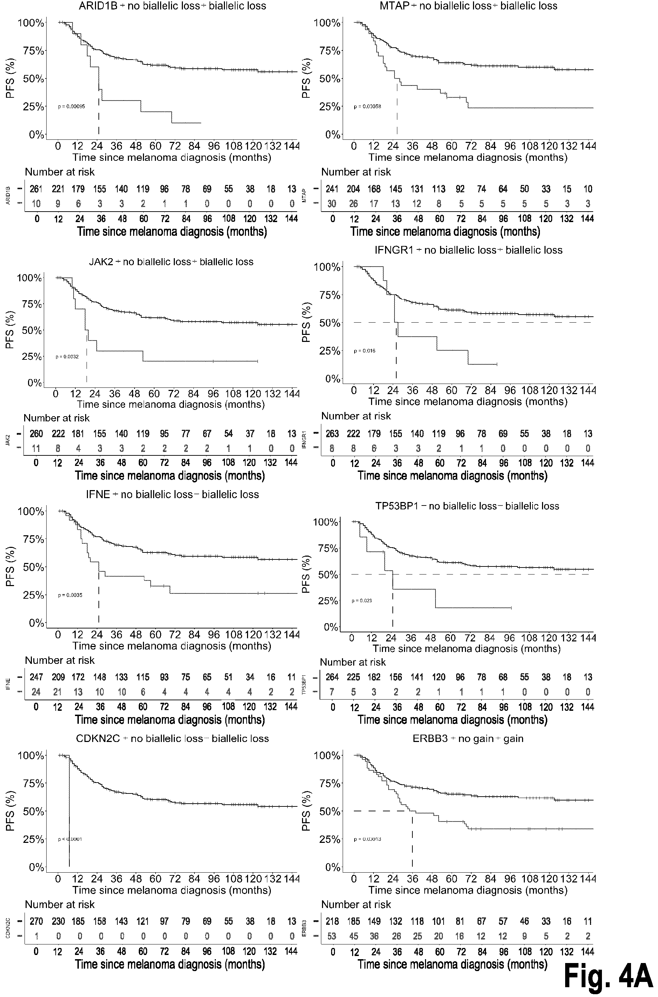

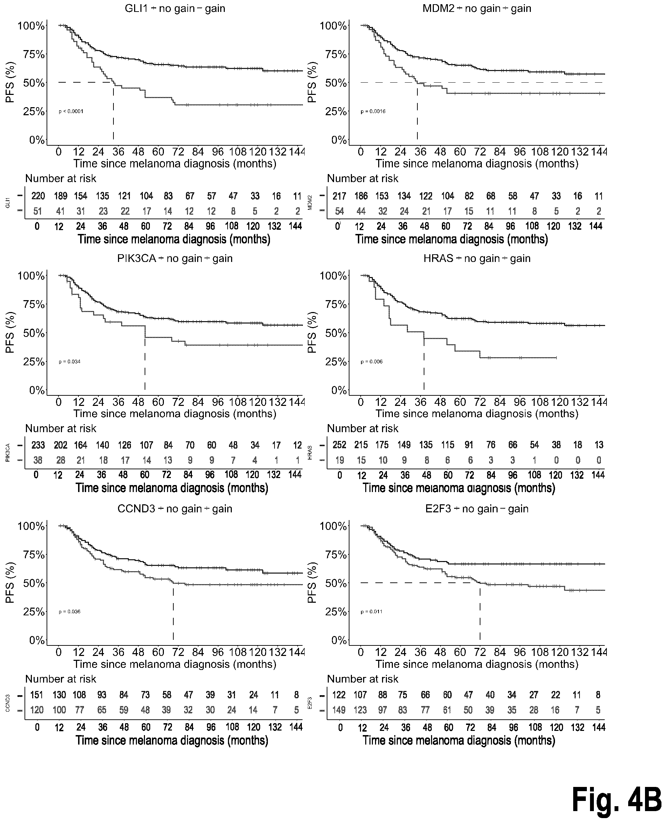

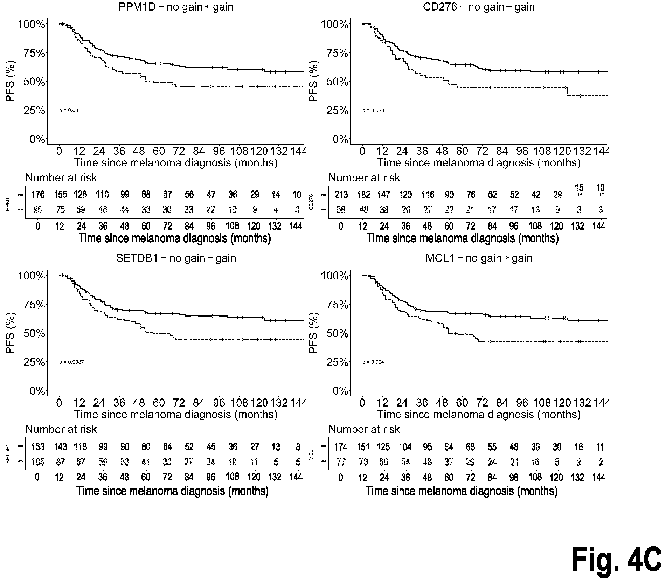

- Fig. 4:: Selected Kaplan Meier curves (A, B, C) for 150-months PFS for specific genomic alterations included in the OncoCycle.

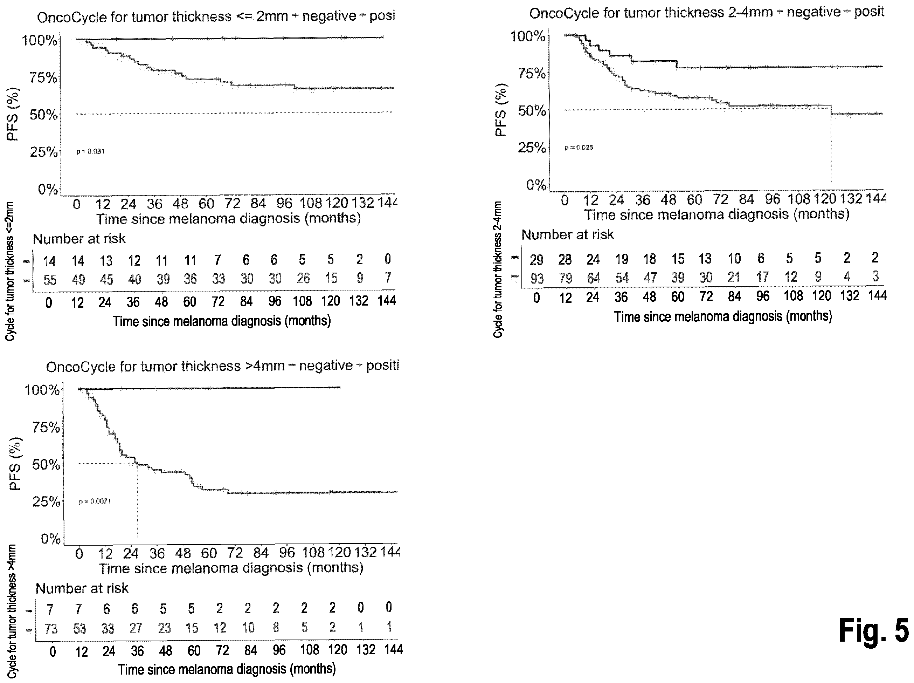

- Fig. 5:: Kaplan Meier curves for the different tumor thickness of primary melanomas stratified according to OncoCycle.

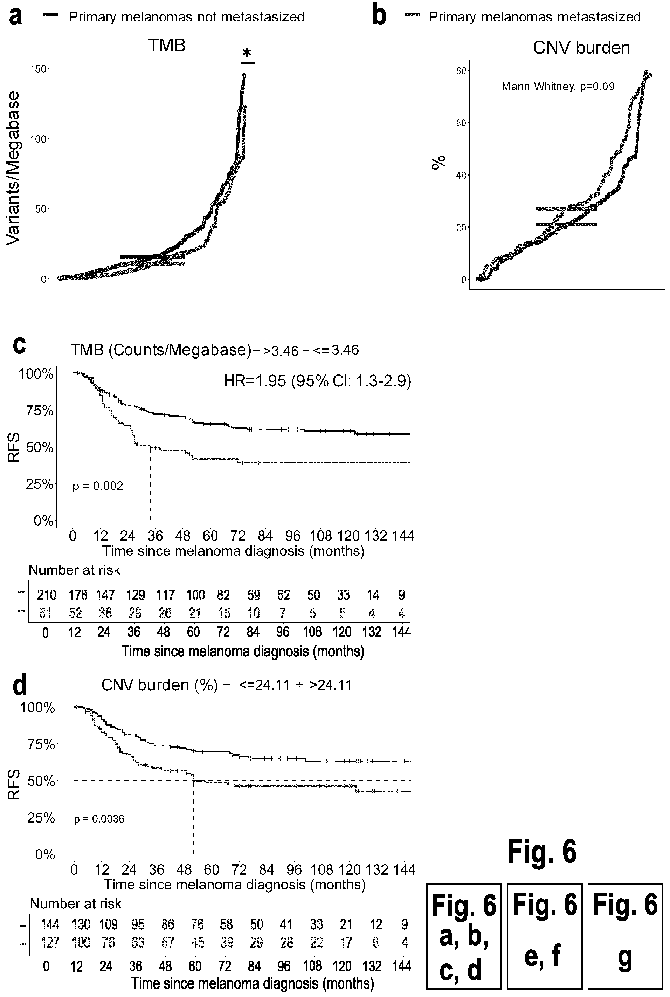

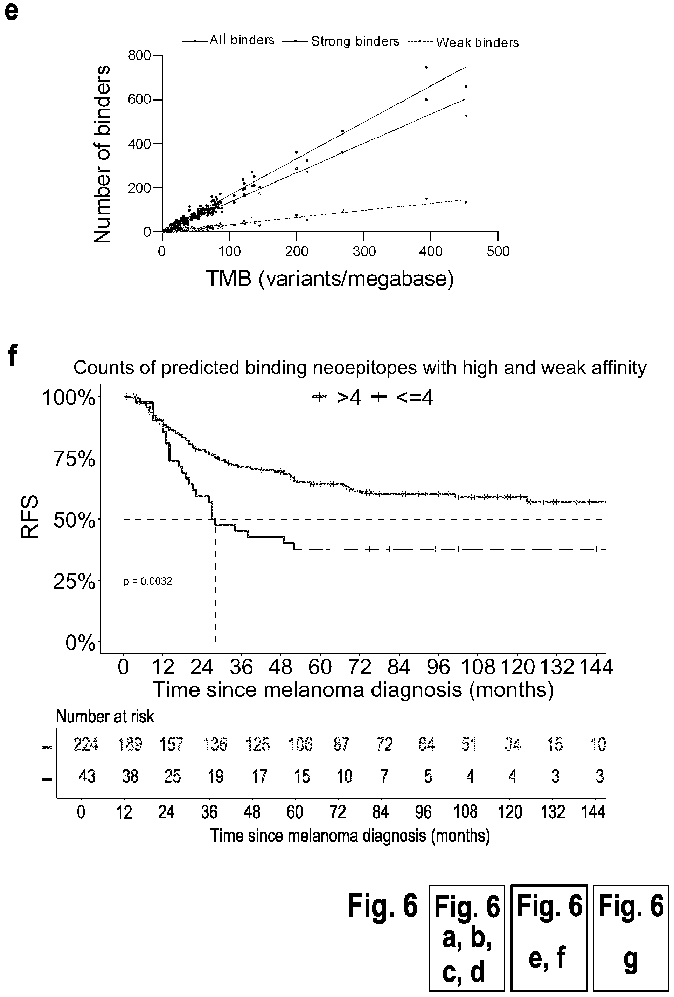

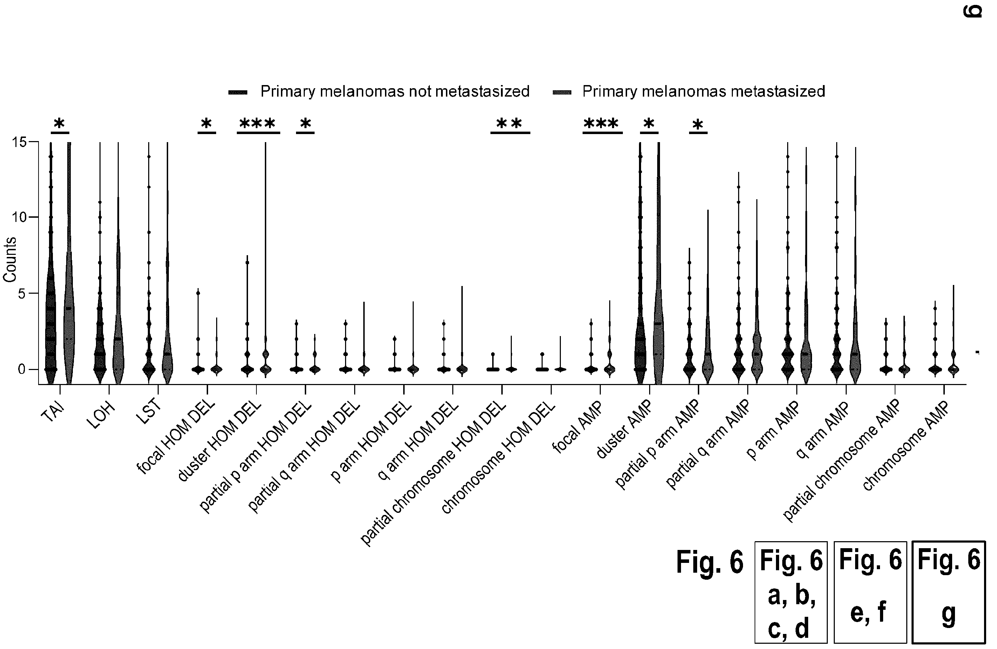

- Fig. 6:: Rank plots showing the differences of primary melanoma between metastasized primaries and not metastasized patients in (a) Tumor mutational burden TMB, (b) single base substitutions SBS, (c) Copy number variant (CNV) burden; Significance is calculated using the Man Whitney test; (d) Violin plots comparing the different types of genomic alterations in primary melanomas between metastasized and non-metastasized patients; Significance was calculated using Mann Whitney test. The significance shown is without multiple comparison correction, after multiple comparison correction only the cluster HOM DEL and focal AMP remain significant, p<0.05;*, p<0.01; **, p<0.001; ***, p<0.0001;****. ; Kaplan-Meier curves showing the 150-month PFS of the 271 patients stratified according to (e) TMB using the optimized cut off of 3.46 (counts/megabase); (f) CNV burden using the optimized cut off of 24.11%; The log rank compares the survival curves for all their follow up time; the Cox regression is for the 150-month PFS.(g) Plot showing the simple linear regression of counts of all, strong and weak binders with TMB in primary melanomas; (h) Kaplan Meier curves showing the 150-month PFS of 271 patients stratified according to their number of predicted binders for PFS using an optimized cut off of 11. The log rank compares the survival curves for all their follow up time; the Cox regression is for the 150-month PFS.

### EXAM PLES

### 1. Patient characteristics

The inventors successfully performed deep DNA sequencing of 708 cancer-related genes and promoter regions, and regions involved in gene fusions of 43 (16%) stage I, 228 (84%) stage II cutaneous melanomas and 70 corresponding metastases of 271 patients prospectively assessed in the German melanoma registry, diagnosed between 2000 and 2018, prior to the establishment of adjuvant therapy. For 271 enrolled CM analyzed, both SNVs and SCNAs assessments were available; 114 (42%) were superficial spreading melanomas (SSM), 62 (23%) nodular melanomas (NM), 46 (16%) acral lentiginous melanomas (ALM), 27 (10%) lentigo maligna melanomas (LMM), 16 (6%) were rarer subtypes, and 6 not specified. Ulceration was documented for 137 (51%) melanomas. Micrometastases were excluded by sentinel lymph node biopsy (SNB) in 225/271 (83%) patients. Overall, 46 (17%) developed only loco-regional metastases (stage III), 42 (15.5%) loco-regional metastases followed by distant metastases, and 22 (8.1%) directly developed distant metastases (in 107 patients metastases occurred within 150 months, and in three later). The median follow up was 95 [95% Cl: 85-105] months, the median progression-free survival (PFS) for the entire cohort was 152 [95% Cl: 90-214] months and median distant metastasis-free survival was 217 [95% Cl: 135-299] months.

Increasing tumor thickness (entire cohort: simple cox: ref: ≤2mm; for 2-4 mm: HR=1.9, 95% Cl: 1.08-3.3, p=0.026; for >4mm: HR=3.5; 95% Cl: 2-6.2, p=0.000012; log rank, p=0.000010) was significantly associated with reduced PFS in the entire cohort; ulceration was significant in the cohort successfully receiving SNB (ulceration: log rank: p=0.043). Tumor thickness and tumor stage predicted the PFS of the cohort (Fig. 1a, b) as established by the AJCC edition 8. Metastases were linked to poor overall survival (log rank test, p<0.0001; Fig. 2).

### 2. Genome signatures predicting reduced PFS of stage I/II CMs

To identify potential genomic alterations that might predispose primary melanomas for progression into metastatic disease, the inventors comprehensively analyzed the 708 cancer associated gene regions using panel sequencing with a 385fold coverage for tumor samples, and 290fold coverage for normal tissue. Within these 708 cancer-associated gene regions, all melanomas had multiple oncogenic mutations resulting from missense, non-sense, non-stop/stop-loss mutations, splice site, transition start site or promoter mutations, frame shift deletions and insertions, and in frame deletions. The most frequently mutated genes include the established mutations in *BRAF*, *NRAS*, *NF1*, *TERT*, and *KIT* with the expected frequency (Fig. 1c). The relatively frequent *KIT* mutations resulted from the large number of *ALM* and *LMM* in this cohort. The median tumor mutational burden (TMB) was 13.3 [IQR: 4.8-33.7] (Fig. 1c). The median number of oncogenic mutations per melanoma was 5 [IQR: 2-10].

Oncogenic single nucleotide variants (SNV) in *BRAF*, *NRAS*, *NF1* occurred in 209/271 (77%), *TERT* (37%) in 99/271 and *KIT* 19/271 (7%) CM (Fig. 1c). CMs had multiple further established oncogenic mutations, the 30 most frequent being shown in the On-coplot (Fig. 1c). In line with previous reports, none of the mutations in these 30 genes associated with an altered PFS in univariate analysis, except oncogenic mutations in NF1 that associated with a lower frequency of metastases.

The inventors identified in all but four CMs oncogenic SCNAs, including amplifications in oncogenes, like *CDK4*, *MYC*, and others or deletions in tumor suppressor genes like *CDKN2A.*

The 21/22/23 SNCAs described in the OncoCycle were significantly associated with worse PFS in univariate COX and significantly associated with metastasis in logistic regression (Fig. 1c). Monoallelic deletions of *CDKN2A* or biallelic losses or gains in the other genes did not shorten the PFS in univariate analysis.

Heat map representation uncovered significant association of distinct chromosomal losses or gains with the occurrence of metastases (Fig. 1d). Also, GISTIC analysis suggested the association of SCNAs in distinct chromosomal regions with the increased risk of developing melanoma metastases.

To determine the pathways associated with these SCNAs, the inventors performed Gene Set Enrichment Analysis (GSEA), by comparing primary melanomas that caused metastases with melanomas that did not. The REACTOME public data base revealed seven out of 109 gene sets at a nominal p value <0.05 in CM that progressed to metastatic disease. Among the four most significant gene sets were cell cycle check points, two TP53-associated gene sets and cellular senescence (Fig. 1e). Also, the Kyoto Encyclopedia of Genes and Genomes (KEGG) pathway dataset, identified signaling of TP53, cell cycle, and bladder cancer as the only significantly enriched pathways out of 44.

In line with the heatmap data and gene set analyses, the inventors identified 21/22/23 SCNAs that significantly associated with the progression of primary CM into metastatic disease in univariate COX analysis (Fig. 3a). Importantly, most of these 21/22/23 genes coded for molecules transmitting interferon (IFN) signaling, molecules controlling cell cycle progression or DNA repair, or molecules controlling chromatin organization (Fig. 3a). One gene amplification coded for an apoptosis inhibitor, *MCL1* and one for *VEGFA*, while other apoptosis-related genes or genes coding for MHC class I recognition were not significant in univariate COX regression (Fig. 3a). In line with the GSEA data, biallelic loss of function in genes coding for either *TP53BP1*, for type I IFNs like *IFNE* or the IFN signaling molecules *IFNGR1, JAK2*, or for chromatin modeling *MTAP, ARID1B,* or the cell cycle inhibitors *CDKN2A,* B, C, or ≥3fold gains of *ERBB3, GLI1, CDK4, MDM2, PIK3CA, HRAS, CCND3, E2F3, PPM1D, SETD81,* CD276, and *MCL1* significantly predicted a strongly reduced PFS (Fig. 3b). Among these 22/23 SCNAs, ≥3fold gains in *CDK4* (Fig. 3c), *ERBB3*, *GLI1* (Fig. 4), and biallelic losses of *CDKN2A*/*B* (Fig. 3c), or of IFN-signalling genes (Fig. 3e), had the strongest impact on the PFS. When combined in the *OncoCycle* genomic biomarker panel (Table 1), these 21/22/23 SCNAs identified more than 94% of primary CM progressing to metastatic disease (Fig. 3b).

**Table 1: The OncoCycle marker genes**

| OncoCycle marker genes | Chromosomal region | Role |
|---|---|---|
| *1&2.CDKN2A*/*B biallelic* | 9p21.3 | Cell cycle regulator, cell cycle arrest in G1 |
| *3.CDKN2C biallelic loss* | 1p32.3 | Cell cycle |
| *4.TP53BP1 biallelic loss* | 15q15.3 | DNA repair protein |
| *5.IFNE biallelic loss* | 9p21.3 | Type I IFNs |
| *6.IFNGR1 biallelic loss* | 6q23.3 | Type II IFNs |
| *7.JAK2 biallelic loss* | 9p24.1 | Type I and II IFNS |
| *8.MTAP biallelic loss* | 9p21.3 | Methionine salvage pathway |
| *9.ARID1B biallelic loss* | 6q25.3 | Transcriptional regulation; chromatine mo- |
| 10.*ERBB3 gain* | 12q13.2 | Receptor tyrosine kinase |
| *11.GLI1 gain* | 12q13.3 | Transcription factor |
| *12.CDK4 gain* | 12q14.1 | Cell cycle regulator |
| *13.MDM2 gain* | 12q15 | Ubiquitin ligase and p53 inhibitor |
| *14.PIK3CA gain* | 3q26.32 | Catalytic subunit of PI3-kinase |
| *15.HRAS gain* | 11p15.5 | GTPase |
| *16.CCND3 gain* | 6p21.1 | Cell cycle regulator |
| *17.E2F3 gain* | 6p22.3 | Cell cycle regulator |
| *18.PPM1D gain* | 17q23.3 | Cell cycle regulator |
| *19.SETDB1 gain* | 1q21.3 | Histone methyltransferase; chromatine mo- |
| *20.CD276 (B7H3) gain* | 15q24.1 | Immune checkpoint molecule |
| *21.MCL1 gain* | 1q21.2 | Apoptosis |
| *22.LAMA2* loss | 6q22.33 | Laminin; senescence control |
| *23.MYC gain* | 8q24.21 | Cell cycle regulator |

Comparing the OncoCycle negative with the OncoCycle positive group in univariate Cox regression analysis, the HR of PFS in the *OncoCycle* positive was 4.9 (95% Cl: 2.1-11.1, p=0.000162). In the multiple Cox regression analysis, the OncoCycle biomarker panel also significantly predicted the development of metastases (Fig. 3b) independent of tumor thickness and ulceration (HR=4.6, 95% Cl: 2-10.5, p=0.000330). Thus, either loss of function or amplifying oncogenic SCNAs in only 22/23 out of 708 genes, analyzed for multiple gene modifications, predicted metastatic disease with high sensitivity. When combined with tumor thickness, the OncoCycle strongly and significantly improved the prognostic value of the TNM classification (Fig. 5).

### 3. Genomic characterization of primary tumors

CMs have a high tumor mutational burden (TMB), with the exception of acral melanomas, and the high TMB of melanoma metastases associates with immune recognition. Unbiased multiparameter analysis revealed the TMB was among the strongest parameters predicting the risk of primary melanomas to progress to metastatic disease. Rank test significantly correlated a high TMB with protection from metastases (Fig. 6a), as did a higher burden in single base substitutions (Fig. 6b). Inversely, a high overall burden in SCNAs associated with an increased incidence of metastases resulting in a reduced PFS (Fig. 6c). When characterizing the SCNAs more in detail, telomeric allelic imbalance (TAI), clusters of homozygous deletions (HOMDEL) and focal amplifications (AMP) significantly correlated with the occurrence of metastases (Fig. 6d), underling that the specific changes in distinct chromosomal areas and the burden in distinct SCNA types are closely associated with CMs prone to develop metastases. In line with this, both, the TMB (cut off 3.46, HR=1.95; 95% Cl: 1.3-2.9; p=0.001), and the CNV burden below 24.11% (HR 1.8; 95% Cl: 1.25 - 2.6; p=0.002) affected the PFS (Fig. 6e, f). In multiple COX regression the OncoCycle, the TMB and the tumor thickness significantly predicted a shortened PFS, with the OncoCycle being the strongest (higher HR) predictor (Table 2).

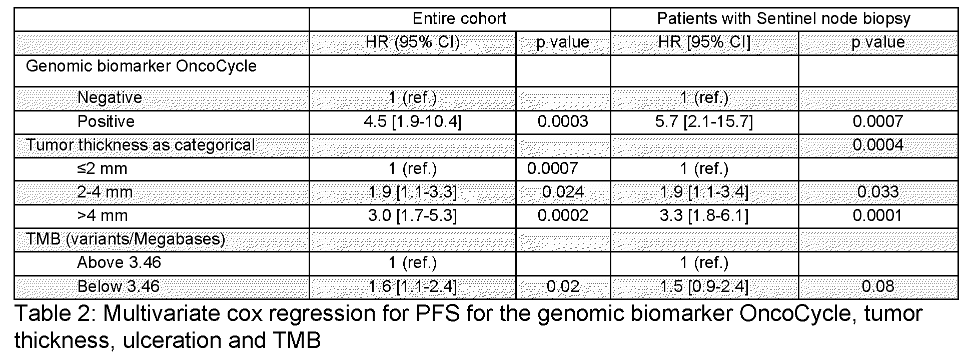

As a high TMB of melanomas results in a high immunogenicity of melanoma metastases, the inventors asked whether the number of HLA-peptide interactions might predict the PFS of primary CMs. In univariate COX regression high numbers of weak and all binders predicted a significantly improved PFS (p=0.013 log rank), and the number of both weak and strong binders correlated significantly with TMB (Fig. 6g, h).

Validation of the panel sequencing with FISH for selected genes, showed that both methods provided comparable data in 90% of the samples, and only 2/33 were really divergent, what may result from intratumoral heterogeneity.

### 6. Conclusion

The inventors provide a method by which the risk of metastasis can be reliably predicted. Since the prediction is possible based on the primary melanoma, it is possible to assess at a very early stage of tumorigenesis whether adjuvant or neo-adjuvant therapy would be advisable. This significantly increases the chances of the patient making a full recovery. Furthermore, patients with a low risk of metastasis can be spared unnecessary adjuvant or neo-adjuvant therapy.

## Claims

1. A method ex *vivo* for classifying a cutaneous melanoma patient, regarding its risk of developing metastases, comprising the following steps:
1. providing a biological sample of the primary cutaneous melanoma,
2. analyzing the biological sample for the presence of a somatic copy number alteration (SCNA) in at least one marker gene,
3. classifying the cutaneous melanoma patient as a high-risk patient if a SCNA is detected in step 2,
wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS, CCND3, E2F3, PPM1D, SETD81,* CD276 *(B7H3), MCL1, LAMA2, and MYC.*

2. The method of any of the preceding claims, wherein the SCNA is an allelic loss and/or a gene amplification.

3. The method of claim 2, wherein the allelic loss is a homozygous (biallelic) loss.

4. The method of claim 2 or 3, wherein the gene amplification is a ≥3fold gene amplification.

5. The method of any of the preceding claims, wherein the following SCNA is detected in the respective marker gene:
| marker gene | SCNA |
|---|---|
| *GLI1* | gene amplification |
| *IFNE* | biallelic loss |
| *IFNGR1* | biallelic loss |
| *JAK2* | biallelic loss |
| *ARID1B* | biallelic loss |
| *CDKN2B* | biallelic loss |
| *CDKN2C* | biallelic loss |
| *TP53BP1* | biallelic loss |
| *ERBB3* | gene amplification |
| *CDK4* | gene amplification |
| *MDM2* | gene amplification |
| *PIK3CA* | gene amplification |
| *HRAS* | gene amplification |
| *CCND3* | gene amplification |
| *E2F3* | gene amplification |
| *PPM1D* | gene amplification |
| *SETDB1* | gene amplification |
| *CD276 (B7H3)* | gene amplification |
| *MCL1* | gene amplification |
| *LAMA2* | biallelic loss |
| *MYC* | gene amplification |

6. The method of any of the preceding claims, wherein at least 2, 3, 4, 5, 6, 7, 8, 9, 10, 11, 12, 13, 14, 15, 16, 17, 18, 19, 20 or all 21 marker genes of the OncoCycle are analyzed.

7. The method of any of the preceding claims, wherein in step 2 at least the following marker genes are analyzed:
- *GLI1, IFNE, IFNGR1, CDK4;* and/or
- *IFNE, IFNGR1, JAK2, CDK4;* and/or
- *GLI1, IFNE, JAK2, ARID1B, ERBB3, CDK4, MCL1, MDM2;* and/or
- *GLI1, IFNE, IFNGR1, ARID1B, ERBB3, CDK4, MCL 1, MDM2, ARID1B, SETD81, HRAS, E2F3,* CD276; and/or
- *GLI1, IFNE, IFNGR 1, JAK2, ARID1B, ERBB3, CDK4, MCL 1, MDM2, SETD81, ARID1B, HRAS, E2F3,* CD276; and/or
- *GLI1, IFNE, IFNGR 1, JAK2, CDKN2B, CDKN2C, TP53BP1, ARID1B, ERBB3, CDK4, MDM2, PIK3CA, HRAS, CCND3, E2F3, PPM1D, SETD81,* CD276 *(B7H3), MCL 1, LAMA2, MYC.*

8. The method of any of the preceding claims, wherein the following additional step(s) is(are) carried out:
2'. analyzing the biological sample for determining the tumor thickness of the primary tumor, and/or
2". analyzing the biological sample for determining the tumor stage, and/or
2‴. analyzing the biological sample for determining the tumor mutational burden (TMB), and/or
2‴ʺ. analyzing the biological sample for determining the copy number variant (CNV) burden.

9. The method of any of the preceding claims, wherein the following additional step is carried out:
3'. prognosing a reduced progression-free survival (PFS) for the patient if a SCNA is detected in step 2.

10. The method of any of the preceding claims, wherein in step (2) the biological sample is analyzed via DNA analysis and/or RNA analysis.

11. The method of any of claims the preceding claims, wherein in step (2) the biological sample is analyzed via Fluorescence in situ hybridization (FISH), and/or via protein analysis.

12. Use of a marker gene which underwent a somatic copy number alteration (SCNA) for classifying a cutaneous melanoma patient, regarding its risk of developing metastases, wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS, CCND3, E2F3, PPM1D, SETD81,* CD276 *(B7H3), MCL1, LAMA2, and MYC.*

13. A method ex *vivo* for identifying treatment targets in a cutaneous melanoma patient, comprising the following steps:
1. providing a biological sample of the primary cutaneous melanoma,
2. analyzing the biological sample for the presence of a somatic copy number alteration (SCNA) in at least one marker gene,
3. identifying a marker gene as a treatment target if a SCNA is detected in step 2,
wherein the marker gene is selected from the group ("OncoCycle") consisting of: *GLI1, IFNE, IFNGR1, JAK2, ARID1B, CDKN2B, CDKN2C, TP53BP1, ERBB3, CDK4, MDM2, PIK3CA, HRAS, CCND3, E2F3, PPM1D, SETD81,* CD276 *(B7H3), MCL1, LAMA2, and MYC.*

14. A method of selecting a treatment of a cutaneous melanoma (CM) patient, comprising the selection of adjuvant or neo-adjuvant therapy, if, after the patient was subjected to the method of claim 13, a marker gene which underwent SCNA was detected in step 3.

15. The method of claim 14, wherein the therapy is selected according to the marker gene as follows:
| Marker gene | Therapy |
|---|---|
| *GLI1* | GLI1 and BET inhibitors, Hedgehog inhibitors, arsenic trioxide |
| *IFNE* | Cytokine therapy and/or cell cycle inhibitors |
| *IFNGR1* | Cell cycle inhibitors, immune therapy secondary |
| *JAK2* | Cell cycle inhibitors, immune therapy secondary |
| *ARID18* | HDAC inhibitors, cell cycle inhibitors, immune checkpoint blockade and/or inhibitors of mTOR, EZH2, histone deacetylases, ATR and/or PARP |
| *CDKN2B* | Cell cycle inhibitors |
| *CDKN2C* | Cell cycle inhibitors |
| *TP53BP1* | Cell cycle inhibitors and/or p53 activators |
| *ERBB3* | ERBB family inhibitors |
| *CDK4* | Cell cycle inhibitors |
| *MDM2* | Cell cycle inhibitors |
| *PIK3CA* | PI3K pathway inhibitors |
| *HRAS* | RAS inhibitors and/or RAS pathway inhibitors |
| *CCND3* | Cell cycle inhibitors |
| *E2F3* | Cell cycle inhibitors |
| *PPM1D* | Cell cycle inhibitors and/or PPM1D inhibitors |
| *SETDB1* | SETDB1 inhibitors, immune checkpoint blockade and/or inhibitors of mTOR, EZH2, histone deacetylases, ATR and/or PARP |
| CD276 *(B7H3)* | Anti-CD276 |
| *MCL1* | MCL1 inhibitors and/or apoptosis promoting drugs |
| *LAMA2* | |
| *MYC* | Cell cycle inhibitors |
